(19) Europäisches Patentamt
European Patent Office
Office européen des brevets

(11) EP 3 992 299 A1

(12) **EUROPÄISCHE PATENTANMELDUNG**

(43) Veröffentlichungstag:
**04.05.2022 Patentblatt 2022/18**

(21) Anmeldenummer: **21204928.2**

(22) Anmeldetag: **27.10.2021**

(51) Internationale Patentklassifikation (IPC):
***C12P 41/00*** *(2006.01)* ***C12N 9/04*** *(2006.01)*
***C12R 1/40*** *(2006.01)*

(52) Gemeinsame Patentklassifikation (CPC):
**C12P 41/002; C12N 9/0006; C12Y 101/01198;**
C12R 2001/40

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **27.10.2020 DE 102020213511**

(71) Anmelder: **Fraunhofer-Gesellschaft zur Förderung der angewandten Forschung e.V.
80686 München (DE)**

(72) Erfinder:
- **Hofer, Michael
70569 Stuttgart (DE)**
- **Kourist, Robert
8044 Graz (AT)**

(74) Vertreter: **Schrell, Andreas et al
Gleiss Große Schrell und Partner mbB
Patentanwälte Rechtsanwälte
Leitzstrasse 45
70469 Stuttgart (DE)**

# (54) VERFAHREN ZUR ENZYMATISCHEN RACEMAT-SPALTUNG VON CAMPHER

(57) Die vorliegende Erfindung betrifft ein Verfahren zur Herstellung einer 1R-(+)-Campher- oder (1S)-(-)-Campher-Enantiomeren-angereicherten Zusammensetzung aus Campher, ein Verfahren zur Herstellung von 1R-(+)-Campher oder (1S)-(-)-Campher aus Campher, eine für diese Verfahren bereitgestellte enantioselektive Borneol-Dehydrogenase, ein Verfahren zur Herstellung von 1R-(-)-Campher-10-Sulfonsäure oder 1S-(+)-Campher-10-Sulfonsäure aus einer 1R-(+)-Campher-Enantiomeren-angereicherten oder 1S-(-)-Campher-Enantiomerenangereicherten Zusammensetzung und ein Verfahren zur Herstellung von 1R-(-)-Campher-10-Sulfonsäure oder 1S-(+)-Campher-10-Sulfonsäure aus 1R-(+)-Campher oder 1S-(-)-Campher.

EP 3 992 299 A1

SEQUENCE LISTING

<110>  Fraunhofer-Gesellschaft

<120>  Verfahren zur enzymatischen Racematspaltung

<130>  111614

<160>  43

<170>  PatentIn version 3.5

<210>  1
<211>  260
<212>  PRT
<213>  Pseudomonas putida ATCC17453

<400>  1

```
Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Glu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175
```

Pro Asp Gly Ile Arg Ile Asn Ala Val Leu Pro Tyr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 2
<211> 783
<212> DNA
<213> Pseudomonas putida ATCC17453

<400> 2
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcggg cttaacaggc gagatcgaca acggcctata tgccgcaacc 480

aaggccgcgg tacatacctg gactcgcaat gtggctcgcc aatgggggcc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 3
<211> 260
<212> PRT
<213> Pseudomonas putida ATCC17453

<400> 3

Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Glu Ala Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Tyr Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
245 250 255

Ile Ala Val Arg
260

<210> 4
<211> 783
<212> DNA
<213> Pseudomonas putida ATCC17453

<400> 4
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcgcg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 5
<211> 260
<212> PRT
<213> Pseudomonas putida ATCC17453

<400> 5

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys

50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Gly Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Tyr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 6
<211> 783
<212> DNA
<213> Pseudomonas putida ATCC17453

<400> 6
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccggctcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 7
<211> 260
<212> PRT
<213> Pseudomonas putida ATCC17453

<400> 7

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr

115 120 125

Ser Leu Met Lys Ser Gln Gly Thr Gly Asn Ile Ile Asn Phe Gly Ser
130 135 140

His Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Tyr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 8
<211> 783
<212> DNA
<213> Pseudomonas putida ATCC17453

<400> 8
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct cccactcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 9
<211> 260
<212> PRT
<213> Pseudomonas putida ATCC17453

<400> 9

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Met Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Tyr Met Val Thr Pro

180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 10
<211> 783
<212> DNA
<213> Pseudomonas putida ATCC17453

<400> 10
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccatgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 11
<211> 260
<212> PRT
<213> Pseudomonas putida ATCC17453

<400> 11

```
Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Leu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Tyr Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
```

245 250 255

Ile Ala Val Arg
260

<210> 12
<211> 783
<212> DNA
<213> Pseudomonas putida ATCC17453

<400> 12
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccctgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 13
<211> 260
<212> PRT
<213> Pseudomonas putida ATCC17453

<400> 13

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Ser Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Tyr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 14
<211> 783
<212> DNA
<213> Pseudomonas putida ATCC17453

<400> 14
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccagctcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc ctacatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 15
<211> 260
<212> PRT
<213> Pseudomonas putida ATCC17453

<400> 15

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
    130                 135                 140

Glu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Leu Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260

<210> 16
<211> 783
<212> DNA
<213> Pseudomonas putida ATCC17453

<400> 16
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cctgatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 17
<211> 260
<212> PRT
<213> Pseudomonas putida ATCC17453

<400> 17

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Glu Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Ile Met Val Thr Pro
180 185 190

```
Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260
```

<210> 18
<211> 783
<212> DNA
<213> Pseudomonas putida ATCC17453

<400> 18

```
atgaaaccgc tagcaggtaa aagaatcatc gtcactggtg gagcacaggg tatcggagcg      60
tcagtcgtgc gcgcctacct ggccgcgggc gcaactgtag tgtcaatgga catgaacgac     120
aaactggggc aacaggtcgt ctccgaggcc attgagaaac accccgactg cagcgcccgc     180
tactgccatt gcgacattac tgatcgcact gcagtggaaa aggttttcgc cgcggcgacc     240
tgggacatgg gcggcctgga cgtcatggtc aacgtagcgg gtgtacagcg ccatagctct     300
ccagacgcca tatcggaaga tctgttcgac ttactgttcc gcgtcaacgt actaggcacc     360
atgaacacca acggcgtggc ctattcgctg atgaaatccc agggcacagg caatatcatc     420
aacttcggct ccgaatcggg cttaacaggc gagatcgaca acggcctata tgccgcaacc     480
aaggccgcgg tacatacctg gactcgcaat gtggctcgcc aatggggccc tgacggcatc     540
cgcatcaatg ccgttctgcc cattatggtc acccctatgt acgtcgactt ccgcaatgcc     600
ctgtcaccag aagcgcttgc ctcccatgac gccgccacca aggccgacat accgctaggc     660
ggaaaatttg gcgatgtcga caaggacttg gcaccagtga tggtcttcct cgccagcgac     720
gcctcccact tcatcaccgg ccagatgttc ccggtagacg gtgggcttat tgcggtgcga     780
tga                                                                   783
```

<210> 19
<211> 260
<212> PRT
<213> Pseudomonas putida ATCC17453

<400> 19

```
Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Glu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Val Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255
```

Ile Ala Val Arg
260

<210> 20
<211> 783
<212> DNA
<213> Pseudomonas putida ATCC17453

<400> 20
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cgtgatggtc accccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 21
<211> 260
<212> PRT
<213> Pseudomonas putida ATCC17453

<400> 21

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Glu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Thr Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260

<210> 22
<211> 783
<212> DNA
<213> Pseudomonas putida ATCC17453

<400> 22
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc caccatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 23
<211> 260
<212> PRT
<213> Pseudomonas putida ATCC17453

<400> 23

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
    130                 135                 140

Glu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Ala Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260

<210> 24
<211> 783
<212> DNA
<213> Pseudomonas putida ATCC17453

<400> 24
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgaatcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cgcgatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 25
<211> 260
<212> PRT
<213> Pseudomonas putida ATCC17453

<400> 25

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Met Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Thr Met Val Thr Pro
180 185 190

```
Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260
```

<210> 26
<211> 783
<212> DNA
<213> Pseudomonas putida ATCC17453

<400> 26

```
atgaaaccgc tagcaggtaa aagaatcatc gtcactggtg gagcacaggg tatcggagcg      60
tcagtcgtgc gcgcctacct ggccgcgggc gcaactgtag tgtcaatgga catgaacgac     120
aaactggggc aacaggtcgt ctccgaggcc attgagaaac accccgactg cagcgcccgc     180
tactgccatt gcgacattac tgatcgcact gcagtggaaa aggttttcgc cgcggcgacc     240
tgggacatgg gcggcctgga cgtcatggtc aacgtagcgg gtgtacagcg ccatagctct     300
ccagacgcca tatcggaaga tctgttcgac ttactgttcc gcgtcaacgt actaggcacc     360
atgaacacca acggcgtggc ctattcgctg atgaaatccc agggcacagg caatatcatc     420
aacttcggct ccatgtcggg cttaacaggc gagatcgaca acggcctata tgccgcaacc     480
aaggccgcgg tacatacctg gactcgcaat gtggctcgcc aatggggccc tgacggcatc     540
cgcatcaatg ccgttctgcc caccatggtc acccctatgt acgtcgactt ccgcaatgcc     600
ctgtcaccag aagcgcttgc ctcccatgac gccgccacca aggccgacat accgctaggc     660
ggaaaatttg gcgatgtcga caaggacttg gcaccagtga tggtcttcct cgccagcgac     720
gcctcccact tcatcaccgg ccagatgttc ccggtagacg gtgggcttat tgcggtgcga     780
tga                                                                   783
```

<210> 27
<211> 260
<212> PRT
<213> Pseudomonas putida ATCC17453

<400> 27

Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Met Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Ala Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
260

<210> 28
<211> 783
<212> DNA
<213> Pseudomonas putida ATCC17453

<400> 28
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccatgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cgcgatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 29
<211> 260
<212> PRT
<213> Pseudomonas putida ATCC17453

<400> 29

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Leu Ser Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Thr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 30
<211> 783
<212> DNA
<213> Pseudomonas putida ATCC17453

<400> 30
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

```
tactgccatt gcgacattac tgatcgcact gcagtggaaa aggttttcgc cgcggcgacc       240

tgggacatgg gcggcctgga cgtcatggtc aacgtagcgg gtgtacagcg ccatagctct       300

ccagacgcca tatcggaaga tctgttcgac ttactgttcc gcgtcaacgt actaggcacc       360

atgaacacca acggcgtggc ctattcgctg atgaaatccc agggcacagg caatatcatc       420

aacttcggct ccctgtcggg cttaacaggc gagatcgaca acggcctata tgccgcaacc       480

aaggccgcgg tacatacctg gactcgcaat gtggctcgcc aatggggccc tgacggcatc       540

cgcatcaatg ccgttctgcc caccatggtc acccctatgt acgtcgactt ccgcaatgcc       600

ctgtcaccag aagcgcttgc ctcccatgac gccgccacca aggccgacat accgctaggc       660

ggaaaatttg gcgatgtcga caaggacttg gcaccagtga tggtcttcct cgccagcgac       720

gcctcccact tcatcaccgg ccagatgttc ccggtagacg gtgggcttat tgcggtgcga       780

tga                                                                      783


<210>  31
<211>  260
<212>  PRT
<213>  Pseudomonas putida ATCC17453

<400>  31

Met Lys Pro Leu Ala Gly Lys Arg Ile Ile Val Thr Gly Gly Ala Gln
1               5                   10                  15


Gly Ile Gly Ala Ser Val Val Arg Ala Tyr Leu Ala Ala Gly Ala Thr
            20                  25                  30


Val Val Ser Met Asp Met Asn Asp Lys Leu Gly Gln Gln Val Val Ser
        35                  40                  45


Glu Ala Ile Glu Lys His Pro Asp Cys Ser Ala Arg Tyr Cys His Cys
    50                  55                  60


Asp Ile Thr Asp Arg Thr Ala Val Glu Lys Val Phe Ala Ala Ala Thr
65                  70                  75                  80


Trp Asp Met Gly Gly Leu Asp Val Met Val Asn Val Ala Gly Val Gln
                85                  90                  95


Arg His Ser Ser Pro Asp Ala Ile Ser Glu Asp Leu Phe Asp Leu Leu
            100                 105                 110


Phe Arg Val Asn Val Leu Gly Thr Met Asn Thr Asn Gly Val Ala Tyr
        115                 120                 125
```

Ser Leu Met Lys Ser Gln Gly Thr Gly Asn Ile Ile Asn Phe Gly Ser
    130                 135                 140

Leu Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Ala Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255

Ile Ala Val Arg
            260

<210> 32
<211> 783
<212> DNA
<213> Pseudomonas putida ATCC17453

<400> 32
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccctgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cgcgatggtc accccctatgt acgtcgactt ccgcaatgcc 600

ctgtcaccag aagcgcttgc ctcccatgac gccgccacca aggccgacat accgctaggc 660

```
ggaaaatttg gcgatgtcga caaggacttg gcaccagtga tggtcttcct cgccagcgac      720

gcctcccact tcatcaccgg ccagatgttc ccggtagacg gtgggcttat tgcggtgcga      780

tga                                                                    783


<210>  33
<211>  260
<212>  PRT
<213>  Pseudomonas putida ATCC17453

<400>  33

Met Lys Pro Leu Ala Gly Lys Arg Ile Ile Val Thr Gly Gly Ala Gln
1               5                   10                  15


Gly Ile Gly Ala Ser Val Val Arg Ala Tyr Leu Ala Ala Gly Ala Thr
            20                  25                  30


Val Val Ser Met Asp Met Asn Asp Lys Leu Gly Gln Gln Val Val Ser
        35                  40                  45


Glu Ala Ile Glu Lys His Pro Asp Cys Ser Ala Arg Tyr Cys His Cys
    50                  55                  60


Asp Ile Thr Asp Arg Thr Ala Val Glu Lys Val Phe Ala Ala Ala Thr
65                  70                  75                  80


Trp Asp Met Gly Gly Leu Asp Val Met Val Asn Val Ala Gly Val Gln
                85                  90                  95


Arg His Ser Ser Pro Asp Ala Ile Ser Glu Asp Leu Phe Asp Leu Leu
            100                 105                 110


Phe Arg Val Asn Val Leu Gly Thr Met Asn Thr Asn Gly Val Ala Tyr
        115                 120                 125


Ser Leu Met Lys Ser Gln Gly Thr Gly Asn Ile Ile Asn Phe Gly Ser
    130                 135                 140


Ser Ser Gly Leu Thr Gly Glu Ile Asp Asn Gly Leu Tyr Ala Ala Thr
145                 150                 155                 160


Lys Ala Ala Val His Thr Trp Thr Arg Asn Val Ala Arg Gln Trp Gly
                165                 170                 175


Pro Asp Gly Ile Arg Ile Asn Ala Val Leu Pro Thr Met Val Thr Pro
            180                 185                 190
```

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 34
<211> 783
<212> DNA
<213> Pseudomonas putida ATCC17453

<400> 34
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccagctcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc caccatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 35
<211> 260
<212> PRT
<213> Pseudomonas putida ATCC17453

<400> 35

Met Lys Pro Leu Ala Gly Lys Arg Ile Ile Val Thr Gly Gly Ala Gln

```
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
65                  70                  75                  80

Trp Asp Met Gly Gly Leu Asp Val Met Val Asn Val Ala Gly Val Gln
                85                  90                  95

Arg His Ser Ser Pro Asp Ala Ile Ser Glu Asp Leu Phe Asp Leu Leu
            100                 105                 110

Phe Arg Val Asn Val Leu Gly Thr Met Asn Thr Asn Gly Val Ala Tyr
        115                 120                 125

Ser Leu Met Lys Ser Gln Gly Thr Gly Asn Ile Ile Asn Phe Gly Ser
    130                 135                 140

Ser Ser Gly Leu Thr Gly Glu Ile Asp Asn Gly Leu Tyr Ala Ala Thr
145                 150                 155                 160

Lys Ala Ala Val His Thr Trp Thr Arg Asn Val Ala Arg Gln Trp Gly
                165                 170                 175

Pro Asp Gly Ile Arg Ile Asn Ala Val Leu Pro Ala Met Val Thr Pro
            180                 185                 190

Met Tyr Val Asp Phe Arg Asn Ala Leu Ser Pro Glu Ala Leu Ala Ser
        195                 200                 205

His Asp Ala Ala Thr Lys Ala Asp Ile Pro Leu Gly Gly Lys Phe Gly
    210                 215                 220

Asp Val Asp Lys Asp Leu Ala Pro Val Met Val Phe Leu Ala Ser Asp
225                 230                 235                 240

Ala Ser His Phe Ile Thr Gly Gln Met Phe Pro Val Asp Gly Gly Leu
                245                 250                 255
```

Ile Ala Val Arg
260

<210> 36
<211> 783
<212> DNA
<213> Pseudomonas putida ATCC17453

<400> 36

```
atgaaaccgc tagcaggtaa aagaatcatc gtcactggtg gagcacaggg tatcggagcg      60

tcagtcgtgc gcgcctacct ggccgcgggc gcaactgtag tgtcaatgga catgaacgac     120

aaactggggc aacaggtcgt ctccgaggcc attgagaaac accccgactg cagcgcccgc     180

tactgccatt gcgacattac tgatcgcact gcagtggaaa aggttttcgc cgcggcgacc     240

tgggacatgg gcggcctgga cgtcatggtc aacgtagcgg gtgtacagcg ccatagctct     300

ccagacgcca tatcggaaga tctgttcgac ttactgttcc gcgtcaacgt actaggcacc     360

atgaacacca acggcgtggc ctattcgctg atgaaatccc agggcacagg caatatcatc     420

aacttcggct ccagctcggg cttaacaggc gagatcgaca acggcctata tgccgcaacc     480

aaggccgcgg tacatacctg gactcgcaat gtggctcgcc aatggggccc tgacggcatc     540

cgcatcaatg ccgttctgcc cgcgatggtc acccctatgt acgtcgactt ccgcaatgcc     600

ctgtcaccag aagcgcttgc ctcccatgac gccgccacca aggccgacat accgctaggc     660

ggaaaatttg gcgatgtcga caaggacttg gcaccagtga tggtcttcct cgccagcgac     720

gcctcccact tcatcaccgg ccagatgttc ccggtagacg gtgggcttat tgcggtgcga     780

tga                                                                   783
```

<210> 37
<211> 260
<212> PRT
<213> Pseudomonas putida ATCC17453

<400> 37

```
Met Lys Pro Leu Ala Gly Lys Arg Ile Ile Val Thr Gly Gly Ala Gln
1               5                   10                  15

Gly Ile Gly Ala Ser Val Val Arg Ala Tyr Leu Ala Ala Gly Ala Thr
            20                  25                  30

Val Val Ser Met Asp Met Asn Asp Lys Leu Gly Gln Gln Val Val Ser
        35                  40                  45

Glu Ala Ile Glu Lys His Pro Asp Cys Ser Ala Arg Tyr Cys His Cys
    50                  55                  60

Asp Ile Thr Asp Arg Thr Ala Val Glu Lys Val Phe Ala Ala Ala Thr
```

65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser
130 135 140

Glu Ala Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Thr Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 38
<211> 783
<212> DNA
<213> Pseudomonas putida ATCC17453

<400> 38
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgcgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc caccatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 39
<211> 260
<212> PRT
<213> Pseudomonas putida ATCC17453

<400> 39

Met Lys Pro Leu Ala Gly Lys Arg Ile Ile Val Thr Gly Gly Ala Gln
1 5 10 15

Gly Ile Gly Ala Ser Val Val Arg Ala Tyr Leu Ala Ala Gly Ala Thr
20 25 30

Val Val Ser Met Asp Met Asn Asp Lys Leu Gly Gln Gln Val Val Ser
35 40 45

Glu Ala Ile Glu Lys His Pro Asp Cys Ser Ala Arg Tyr Cys His Cys
50 55 60

Asp Ile Thr Asp Arg Thr Ala Val Glu Lys Val Phe Ala Ala Ala Thr
65 70 75 80

Trp Asp Met Gly Gly Leu Asp Val Met Val Asn Val Ala Gly Val Gln
85 90 95

Arg His Ser Ser Pro Asp Ala Ile Ser Glu Asp Leu Phe Asp Leu Leu
100 105 110

Phe Arg Val Asn Val Leu Gly Thr Met Asn Thr Asn Gly Val Ala Tyr
115 120 125

Ser Leu Met Lys Ser Gln Gly Thr Gly Asn Ile Ile Asn Phe Gly Ser

130 135 140

Glu Ala Gly Leu Thr Gly Glu Ile Asp Asn Gly Leu Tyr Ala Ala Thr
145 150 155 160

Lys Ala Ala Val His Thr Trp Thr Arg Asn Val Ala Arg Gln Trp Gly
165 170 175

Pro Asp Gly Ile Arg Ile Asn Ala Val Leu Pro Ala Met Val Thr Pro
180 185 190

Met Tyr Val Asp Phe Arg Asn Ala Leu Ser Pro Glu Ala Leu Ala Ser
195 200 205

His Asp Ala Ala Thr Lys Ala Asp Ile Pro Leu Gly Gly Lys Phe Gly
210 215 220

Asp Val Asp Lys Asp Leu Ala Pro Val Met Val Phe Leu Ala Ser Asp
225 230 235 240

Ala Ser His Phe Ile Thr Gly Gln Met Phe Pro Val Asp Gly Gly Leu
245 250 255

Ile Ala Val Arg
260

<210> 40
<211> 783
<212> DNA
<213> Pseudomonas putida ATCC17453

<400> 40
atgaaaccgc tagcaggtaa aagaatcatc gtcactggtg gagcacaggg tatcggagcg 60

tcagtcgtgc gcgcctacct ggccgcgggc gcaactgtag tgtcaatgga catgaacgac 120

aaactggggc aacaggtcgt ctccgaggcc attgagaaac accccgactg cagcgcccgc 180

tactgccatt gcgacattac tgatcgcact gcagtggaaa aggttttcgc cgcggcgacc 240

tgggacatgg gcggcctgga cgtcatggtc aacgtagcgg gtgtacagcg ccatagctct 300

ccagacgcca tatcggaaga tctgttcgac ttactgttcc gcgtcaacgt actaggcacc 360

atgaacacca acggcgtggc ctattcgctg atgaaatccc agggcacagg caatatcatc 420

aacttcggct ccgcgtcggg cttaacaggc gagatcgaca acggcctata tgccgcaacc 480

aaggccgcgg tacatacctg gactcgcaat gtggctcgcc aatggggccc tgacggcatc 540

cgcatcaatg ccgttctgcc cgcgatggtc acccctatgt acgtcgactt ccgcaatgcc 600

ctgtcaccag aagcgcttgc ctcccatgac gccgccacca aggccgacat accgctaggc 660

ggaaaatttg gcgatgtcga caaggacttg gcaccagtga tggtcttcct cgccagcgac 720

gcctcccact tcatcaccgg ccagatgttc ccggtagacg gtgggcttat tgcggtgcga 780

tga 783

<210> 41
<211> 6117
<212> DNA
<213> Pseudomonas putida ATCC17453

<400> 41
gatgcaccac caccaccacc acaaaccgct agcaggtaaa agaatcatcg tcactggtgg 60

agcacagggt atcggagcgt cagtcgtgcg cgcctacctg gccgcgggcg caactgtagt 120

gtcaatggac atgaacgaca aactggggca acaggtcgtc tccgaggcca ttgagaaaca 180

ccccgactgc agcgcccgct actgccattg cgacattact gatcgcactg cagtggaaaa 240

ggttttcgcc gcggcgacct gggacatggg cggcctggac gtcatggtca acgtagcggg 300

tgtacagcgc catagctctc cagacgccat atcggaagat ctgttcgact tactgttccg 360

cgtcaacgta ctaggcacca tgaacaccaa cggcgtggcc tattcgctga tgaaatccca 420

gggcacaggc aatatcatca acttcggctc cgaatcgggc ttaacaggcg agatcgacaa 480

cggcctatat gccgcaacca aggccgcggt acatacctgg actcgcaatg tggctcgcca 540

atggggccct gacggcatcc gcatcaatgc cgttctgccc tacatggtca cccctatgta 600

cgtcgacttc cgcaatgccc tgtcaccaga agcgcttgcc tcccatgacg ccgccaccaa 660

ggccgacata ccgctaggcg gaaaatttgg cgatgtcgac aaggacttgg caccagtgat 720

ggtcttcctc gccagcgacg cctcccactt catcaccggc cagatgttcc cggtagacgg 780

tgggcttatt gcggtgcgat gagtacggcc ccgcccctga ggaggcaata tgttcttttc 840

aaaggaaaag caaacggcaa tcgattgtct ggttgagcgg ccgcactcga gcaccaccac 900

caccaccact gagatccggc tgctaacaaa gcccgaaagg aagctgagtt ggctgctgcc 960

accgctgagc aataactagc ataacccctt ggggcctcta aacgggtctt gaggggtttt 1020

ttgctgaaag gaggaactat atccggattg gcgaatggga cgcgccctgt agcggcgcat 1080

taagcgcggc gggtgtggtg gttacgcgca gcgtgaccgc tacacttgcc agcgccctag 1140

cgcccgctcc tttcgctttc ttcccttcct ttctcgccac gttcgccggc tttccccgtc 1200

aagctctaaa tcgggggctc cctttagggt tccgatttag tgctttacgg cacctcgacc 1260

ccaaaaaact tgattagggt gatggttcac gtagtgggcc atcgccctga tagacggttt 1320

ttcgcccttt gacgttggag tccacgttct ttaatagtgg actcttgttc caaactggaa 1380

caacactcaa ccctatctcg gtctattctt ttgatttata agggattttg ccgatttcgg 1440

cctattggtt aaaaaatgag ctgatttaac aaaaatttaa cgcgaatttt aacaaaatat 1500

taacgtttac aatttcaggt ggcacttttc ggggaaatgt gcgcggaacc cctatttgtt 1560
tatttttcta aatacattca aatatgtatc cgctcatgaa ttaattctta gaaaaactca 1620
tcgagcatca aatgaaactg caatttattc atatcaggat tatcaatacc atatttttga 1680
aaaagccgtt tctgtaatga aggagaaaac tcaccgaggc agttccatag gatggcaaga 1740
tcctggtatc ggtctgcgat tccgactcgt ccaacatcaa tacaacctat taatttcccc 1800
tcgtcaaaaa taaggttatc aagtgagaaa tcaccatgag tgacgactga atccggtgag 1860
aatggcaaaa gtttatgcat ttctttccag acttgttcaa caggccagcc attacgctcg 1920
tcatcaaaat cactcgcatc aaccaaaccg ttattcattc gtgattgcgc ctgagcgaga 1980
cgaaatacgc gatcgctgtt aaaaggacaa ttacaaacag gaatcgaatg caaccggcgc 2040
aggaacactg ccagcgcatc aacaatattt tcacctgaat caggatattc ttctaatacc 2100
tggaatgctg ttttcccggg gatcgcagtg gtgagtaacc atgcatcatc aggagtacgg 2160
ataaaatgct tgatggtcgg aagaggcata aattccgtca gccagtttag tctgaccatc 2220
tcatctgtaa catcattggc aacgctacct ttgccatgtt tcagaaacaa ctctggcgca 2280
tcgggcttcc catacaatcg atagattgtc gcacctgatt gcccgacatt atcgcgagcc 2340
catttatacc catataaatc agcatccatg ttggaattta atcgcggcct agagcaagac 2400
gtttcccgtt gaatatggct cataacaccc cttgtattac tgtttatgta agcagacagt 2460
tttattgttc atgaccaaaa tcccttaacg tgagttttcg ttccactgag cgtcagaccc 2520
cgtagaaaag atcaaaggat cttcttgaga tccttttttt ctgcgcgtaa tctgctgctt 2580
gcaaacaaaa aaaccaccgc taccagcggt ggtttgtttg ccggatcaag agctaccaac 2640
tctttttccg aaggtaactg gcttcagcag agcgcagata ccaaatactg tccttctagt 2700
gtagccgtag ttaggccacc acttcaagaa ctctgtagca ccgcctacat acctcgctct 2760
gctaatcctg ttaccagtgg ctgctgccag tggcgataag tcgtgtctta ccgggttgga 2820
ctcaagacga tagttaccgg ataaggcgca gcggtcgggc tgaacggggg gttcgtgcac 2880
acagcccagc ttggagcgaa cgacctacac cgaactgaga tacctacagc gtgagctatg 2940
agaaagcgcc acgcttcccg aagggagaaa ggcggacagg tatccggtaa gcggcagggt 3000
cggaacagga gagcgcacga gggagcttcc agggggaaac gcctggtatc tttatagtcc 3060
tgtcgggttt cgccacctct gacttgagcg tcgatttttg tgatgctcgt caggggggcg 3120
gagcctatgg aaaaacgcca gcaacgcggc ctttttacgg ttcctggcct tttgctggcc 3180
ttttgctcac atgttctttc ctgcgttatc ccctgattct gtggataacc gtattaccgc 3240
ctttgagtga gctgataccg ctcgccgcag ccgaacgacc gagcgcagcg agtcagtgag 3300
cgaggaagcg gaagagcgcc tgatgcggta ttttctcctt acgcatctgt gcggtatttc 3360
acaccgcata tatggtgcac tctcagtaca atctgctctg atgccgcata gttaagccag 3420

tatacactcc gctatcgcta cgtgactggg tcatggctgc gccccgacac ccgccaacac 3480

ccgctgacgc gccctgacgg gcttgtctgc tcccggcatc cgcttacaga caagctgtga 3540

ccgtctccgg gagctgcatg tgtcagaggt tttcaccgtc atcaccgaaa cgcgcgaggc 3600

agctgcggta aagctcatca gcgtggtcgt gaagcgattc acagatgtct gcctgttcat 3660

ccgcgtccag ctcgttgagt ttctccagaa gcgttaatgt ctggcttctg ataaagcggg 3720

ccatgttaag ggcggttttt tcctgtttgg tcactgatgc ctccgtgtaa gggggatttc 3780

tgttcatggg ggtaatgata ccgatgaaac gagagaggat gctcacgata cgggttactg 3840

atgatgaaca tgcccggtta ctggaacgtt gtgagggtaa acaactggcg gtatggatgc 3900

ggcgggacca gagaaaaatc actcagggtc aatgccagcg cttcgttaat acagatgtag 3960

gtgttccaca gggtagccag cagcatcctg cgatgcagat ccggaacata atggtgcagg 4020

gcgctgactt ccgcgtttcc agactttacg aaacacggaa accgaagacc attcatgttg 4080

ttgctcaggt cgcagacgtt ttgcagcagc agtcgcttca cgttcgctcg cgtatcggtg 4140

attcattctg ctaaccagta aggcaacccc gccagcctag ccgggtcctc aacgacagga 4200

gcacgatcat gcgcacccgt ggggccgcca tgccggcgat aatggcctgc ttctcgccga 4260

aacgtttggt ggcgggacca gtgacgaagg cttgagcgag ggcgtgcaag attccgaata 4320

ccgcaagcga caggccgatc atcgtcgcgc tccagcgaaa gcggtcctcg ccgaaaatga 4380

cccagagcgc tgccggcacc tgtcctacga gttgcatgat aaagaagaca gtcataagtg 4440

cggcgacgat agtcatgccc cgcgcccacc ggaaggagct gactgggttg aaggctctca 4500

agggcatcgg tcgagatccc ggtgcctaat gagtgagcta acttacatta attgcgttgc 4560

gctcactgcc cgctttccag tcgggaaacc tgtcgtgcca gctgcattaa tgaatcggcc 4620

aacgcgcggg gagaggcggt ttgcgtattg ggcgccaggg tggtttttct tttcaccagt 4680

gagacgggca acagctgatt gcccttcacc gcctggccct gagagagttg cagcaagcgg 4740

tccacgctgg tttgccccag caggcgaaaa tcctgtttga tggtggttaa cggcgggata 4800

taacatgagc tgtcttcggt atcgtcgtat cccactaccg agatatccgc accaacgcgc 4860

agcccggact cggtaatggc gcgcattgcg cccagcgcca tctgatcgtt ggcaaccagc 4920

atcgcagtgg gaacgatgcc ctcattcagc atttgcatgg tttgttgaaa accggacatg 4980

gcactccagt cgccttcccg ttccgctatc ggctgaattt gattgcgagt gagatattta 5040

tgccagccag ccagacgcag acgcgccgag acagaactta atgggcccgc taacagcgcg 5100

atttgctggt gacccaatgc gaccagatgc tccacgccca gtcgcgtacc gtcttcatgg 5160

gagaaaataa tactgttgat gggtgtctgg tcagagacat caagaaataa cgccggaaca 5220

ttagtgcagg cagcttccac agcaatggca tcctggtcat ccagcggata gttaatgatc 5280

```
agcccactga cgcgttgcgc gagaagattg tgcaccgccg ctttacaggc ttcgacgccg     5340

cttcgttcta ccatcgacac caccacgctg gcacccagtt gatcggcgcg agatttaatc     5400

gccgcgacaa tttgcgacgg cgcgtgcagg gccagactgg aggtggcaac gccaatcagc     5460

aacgactgtt tgcccgccag ttgttgtgcc acgcggttgg gaatgtaatt cagctccgcc     5520

atcgccgctt ccactttttc ccgcgttttc gcagaaacgt ggctggcctg gttcaccacg     5580

cgggaaacgg tctgataaga gacaccggca tactctgcga catcgtataa cgttactggt     5640

ttcacattca ccaccctgaa ttgactctct tccgggcgct atcatgccat accgcgaaag     5700

gttttgcgcc attcgatggt gtccgggatc tcgacgctct cccttatgcg actcctgcat     5760

taggaagcag cccagtagta ggttgaggcc gttgagcacc gccgccgcaa ggaatggtgc     5820

atgcaaggag atggcgccca acagtccccc ggccacgggg cctgccacca tacccacgcc     5880

gaaacaagcg ctcatgagcc cgaagtggcg agcccgatct tccccatcgg tgatgtcggc     5940

gatataggcg ccagcaaccg cacctgtggc gccggtgatg ccggccacga tgcgtccggc     6000

gtagaggatc gagatctcga tcccgcgaaa ttaatacgac tcactatagg ggaattgtga     6060

gcggataaca attcccctct agaaataatt ttgtttaact ttaagaagga gatatac       6117
```

```
<210>  42
<211>  58
<212>  DNA
<213>  Pseudomonas putida ATCC17453

<400>  42
gatcccatgg atgaaaccgc tagcagggat agcggccgct caaccagaca atcgattg         58


<210>  43
<211>  82
<212>  DNA
<213>  Pseudomonas putida ATCC17453

<400>  43
ttggtctcgg atgcaccacc accaccacca caaaccgcta gcaggtaaaa ggatagcggc       60

cgctcaacca gacaatcgat tg                                                82
```

## Beschreibung

Die vorliegende Erfindung betrifft ein Verfahren zur Herstellung einer 1R-(+)-Campher- oder 1S-(-)-Campher-Enantiomeren-angereicherten Zusammensetzung aus Campher, ein Verfahren zur Herstellung von 1R-(+)-Campher oder 1S-(-)-Campher aus Campher, eine für diese Verfahren bereitgestellte enantioselektive Borneol-Dehydrogenase, ein Verfahren zur Herstellung von 1R-(-)-Campher-10-Sulfonsäure oder 1S-(+)-Campher-10-Sulfonsäure aus einer 1R-(+)-Campher-Enantiomeren-angereicherten oder 1S-(-)-Campher-Enantiomeren-angereicherten Zusammensetzung und ein Verfahren zur Herstellung von 1R-(-)-Campher-10-Sulfonsäure oder 1S-(+)-Campher-10-Sulfonsäure aus 1R-(+)-Campher oder 1S-(-)-Campher.

Terpene stellen eine der größten Gruppen unter den Naturstoffen dar. Diese sind Kohlenwasserstroffe, die anhand ihrer Anzahl von Isopreneinheiten, umfassend Hemiterpene (eine Isopreneinheit) bis Polyterpene (mehr als acht Isopreneinheiten), klassifiziert werden. Monoterpene sind Dimere des Isoprens und können in azyklische, monozyklische, bizyklische und trizyklische Verbindungen unterteilt werden. Monoterpen-Derivate, die Sauerstoff oder Stickstoff aufweisen, werden als Monoterpenoide bezeichnet. Campher ist ein bizyklisches Sauerstoff-aufweisendes Monoterpenoid. In der Natur wird es durch Pflanzen hergestellt (Banthorpe et al., Sogo Kango 72, 1972, 115-155) und von Mikroorganismen wie P. putida (Bradshaw et al., JACS, 81, 1959, 788-824) abgebaut. Pflanzen können sowohl (+)- als auch (-)-Campher synthetisieren, wobei nur der (+)-Campher des Campherbaums wirtschaftlich gewonnen werden kann. (-)-Campher ist über ein kostenintensives Verfahren, einer Oxidation des (-)-Borneols, zugänglich. Des Weiteren kann racemischer Campher kostengünstig aus $\alpha$-Pinen hergestellt werden, welches als Nebenprodukt der Zellstoff- und Papierindustrie erhalten wird (Zhen-Dong, Liang-Wu, Chemical Engineering, 43, 2009, 1-8). Die Nachfrage speziell von (-)-Campher, der für die Synthese von Pharmazeutika eingesetzt wird, steigt stetig (Xu et al., Neurosci., 25, 2005, 8924-8937; Selescu et al., Chem. Sense, 38, 2013; 563-575; Sherkheli et al., Pak. J.. Pharm. Sci., 26, 2013, 431-438). Des Weiteren wird Campher zur Herstellung von Campher-10-Sulfonsäure eingesetzt, wobei racemisches Campher als Edukt verwendet wird und die dadurch erhaltene racemische Campher-10-Sulfonsäure nachteilig durch aufwändige Aufreinigungsschritte, offenbart in der Patentschrift US 3,819,689 A oder in einer weiteren Patentschrift IN 151660 A1, getrennt werden muss. Aus diesem Grund ist es wünschenswert, direkt einen nicht-racemischen Campher einzusetzen und demnach einen einfachen und effizienten Ansatz zur Trennung von racemischem Campher zu entwickeln. Enzymatisch kinetische Resolution (Racematspaltung) ist eine gängige Methode zur Auftrennung von racemischen Gemischen (Breuer et al., Angew. Chem. Int., 43, 2004, 788-824; Verho und Backvall, JACS, 137, 2015, 3996-4006). Alkohol-Dehydrogenasen werden zur Oxidation von Alkoholen sowie deren Reduktion verwendet. Für Campher wurde jedoch allein die 5-Exo-Hydroxy-Campher-Dehydrogenase detailliert beschrieben (Hofer et al., ChemCatChem, 5, 2013, 3351-3357). Es sind jedoch einige Borneol-Dehydrogenasen (BDH) bekannt, zum Beispiel eine selektive (+)-Borneol-Dehydrogenase aus Salvia officinalis oder eine selektive (-)-Borneol-Dehydrogenase aus Tanacetum vulgare (Dehal, Croteau, Arch. Biochem. Biophys., 258, 1987, 287-291; Drienovská et al., Phytochemistry, 172, 2020, 112227). Diese zeigen nachteiligerweise nur eine geringe Aktivität. Des Weiteren sind verschiedene nicht-selektive Alkohol-Dehydrogenasen bekannt, insbesondere die von Tsang (Tsang et al., Appl. Environ. Microbiol., 82, 2016, 6378-6385) offenbarte nicht-stereoselektive Borneol-Dehydrogenase aus Pseudomonas sp., die die höchste Aktivität der bisher bekannten Borneol-Dehydrogenasen aufweist.

Es ist keine Borneol-Dehydrogenase im Stand der Technik offenbart, die zur Reduktion von Campher befähigt ist.

Das der vorliegenden Erfindung zugrundeliegende technische Problem liegt daher darin, die vorgenannten Nachteile zu überwinden. Insbesondere liegt das der vorliegenden Erfindung zugrundeliegende technische Problem darin, Verfahren und Mittel bereitzustellen, die es ermöglichen, in einem Gemisch von Campher-Enantiomeren, insbesondere racemischem Campher, eine Racematspaltung durchzuführen, insbesondere enantioselektiv ein gewünschtes Enantiomer des Camphers anzureichern, insbesondere in reiner Form zu gewinnen. Insbesondere ist es ein technisches Problem der vorliegenden Erfindung, Verfahren und Mittel bereitzustellen, um aus einem racemischem Campher-Gemisch eine Enantiomeren-angereicherte Zusammensetzung, insbesondere Enantiomeren-reines 1R-(+)-Campher und/oder 1S-(-)-Campher zu gewinnen. Insbesondere ist auch ein technisches Problem der vorliegenden Erfindung Verfahren und Mittel bereitzustellen, die die Bereitstellung von Enantiomeren-angereicherter, insbesondere Enantiomeren-reiner, 1R-(-)-Campher-10-Sulfonsäure und/oder 1S-(+)-Campher-10-Sulfonsäure ermöglichen. Darüber hinaus ist ein technisches Problem der vorliegenden Erfindung, derartige Verfahren und Mittel bereitzustellen, die kostengünstig und leicht handhabbar sind.

Das technische Problem wird durch die Lehre der vorliegenden Erfindung gelöst, insbesondere durch ein Verfahren zur Herstellung einer 1R-(+)-Campher- oder 1S-(-)-Campher-Enantiomeren-angereicherten Zusammensetzung aus Campher, umfassend die folgenden Verfahrensschritte:

a) Bereitstellen von Campher, einem Reaktionsmedium und einer enantioselektiven Borneol-Dehydrogenase,
b) Umsetzen des in Verfahrens schritt a) bereitgestellten Camphers in dem Reaktionsmedium mit der enantioselektiven Borneol-Dehydrogenase und

c) Erhalten einer 1R-(+)-Campher-Enantiomeren-angereicherten Zusammensetzung umfassend 1R-(+)-Campher und (-)-Borneol oder einer 1S-(-)-Campher-Enantiomeren-angereicherten Zusammensetzung umfassend 1S-(-)-Campher und (+)-Borneol.

In bevorzugter Ausführungsform der vorliegenden Erfindung wird das technische Problem gelöst durch ein Verfahren zur Herstellung einer 1S-(-)-Campher-Enantiomeren-angereicherten Zusammensetzung aus Campher, umfassend die folgenden Verfahrensschritte:

a) Bereitstellen von Campher, einem Reaktionsmedium und einer enantioselektiven (+)-Borneol-Dehydrogenase,

b) Umsetzen des in Verfahrens schritt a) bereitgestellten Camphers in dem Reaktionsmedium mit der enantioselektiven (+)-Borneol-Dehydrogenase und

c) Erhalten einer 1S-(-)-Campher-Enantiomeren-angereicherten Zusammensetzung umfassend 1S-(-)-Campher und (+)-Borneol.

Erfindungsgemäß ist demgemäß vorgesehen, in einem ersten Verfahrensschritt a) Campher und eine enantioselektive Borneol-Dehydrogenase sowie ein Reaktionsmedium bereitzustellen. Im Rahmen eines zweiten Verfahrensschritts b) wird in dem Reaktionsmedium Campher mit der enantioselektiven Borneol-Dehydrogenase umgesetzt, wobei dadurch vorteilhafterweise eine enantioselektive Umsetzung, insbesondere Reduktion, des Camphers erfolgt. In dem so umgesetzten Reaktionsgemisch wird gemäß Verfahrensschritt c) eine 1R-(+)-Campher-Enantiomeren-angereicherte Zusammensetzung umfassend 1R-(+)-Campher und (-)-Borneol oder eine 1S-(-)-Campher-Enantiomeren-angereicherte Zusammensetzung umfassend 1S-(-)-Campher und (+)-Borneol erhalten.

In bevorzugter Ausführung der vorliegenden Erfindung ist das in Verfahrensschritt b) vorgesehene Umsetzen des im Verfahrensschritt a) bereitgestellten Camphers eine Reduktion. Die Erfindung ermöglicht es, auf diese Weise aus einem Ausgangsmaterial, nämlich Campher, in enantioselektiver Weise eine Enantiomeren-angereicherte Zusammensetzung bereitzustellen, insbesondere eine 1R-(+)-Campher-Enantiomeren-angereicherte Zusammensetzung und eine 1S-(-)-Campher-Enantiomeren-angereicherte Zusammensetzung, insbesondere ermöglicht sie es, 1R-(+)-Campher Enantiomeren-rein und 1S-(-)-Campher Enantiomeren-rein herzustellen. Die vorliegende Erfindung stellt zu diesem Zweck eine enantioselektive Borneol-Dehydrogenase (auch als BDH bezeichnet), insbesondere (+)-Borneol-Dehydrogenase, bereit, die in der Lage ist, Campher zu reduzieren. Die erfindungsgemäß bereitgestellte und eingesetzte enantioselektive Borneol-Dehydrogenase, insbesondere (+)-Borneol-Dehydrogenase, kann demgemäß vorteilhafterweise zur Racematspaltung von Campher eingesetzt werden. Erfindungsgemäß wird daher eine besonders einfache, leicht handhabbare und kostengünstige Enantiomeren-Trennung von Campher ermöglicht, sodass ein hoher apparativer und kostenbezogener Aufwand vermieden wird. Die vorliegende Erfindung ist auch insofern vorteilhaft, als dass sie die sich häufig an eine Campher-Bereitstellung anschließende Sulfonierung von Campher zur Herstellung von Campher-10-Sulfonsäure in enantioselektiver Weise ermöglicht, und demgemäß die Herstellung von 1R-(-)-Campher-10-Sulfonsäure und 1S-(+)-Campher-10-Sulfonsäure ermöglicht, ohne dass kostenintensive und umfangreiche Aufreinigungsschritte durchgeführt werden müssten.

Erfindungsgemäß wird Campher mittels der enantioselektiven Borneol-Dehydrogenase, insbesondere (+)-Borneol-Dehydrogenase, so umgesetzt, dass ein Gemisch aus 1R-(+)-Campher und (-)-Borneol oder ein Gemisch aus 1S-(-)-Campher und (+)-Borneol in Abhängigkeit von der Enantioselektivität der eingesetzten enantioselektiven Borneol-Dehydrogenase erhalten wird.

Die erfindungsgemäß eingesetzte enantioselektive Borneol-Dehydrogenase, insbesondere (+)-Borneol-Dehydrogenase, zeichnet sich insbesondere dadurch aus, dass sie eine Aminosäuresequenz aufweist, die der Wildtyp-Aminosäuresequenz einer Borneol-Dehydrogenase aus pflanzlichen oder mikrobiellen Quellen gleicht, das heißt identisch ist bis auf mindestens einen Aminosäure-Austausch in dieser Aminosäuresequenz und zwar derartig, dass die Aminosäuresequenz der erfindungsgemäßen Borneol-Dehydrogenase, insbesondere (+)-Borneol-Dehydrogenase, gegenüber der Aminosäuresequenz der Wildtyp-Borneol-Dehydrogenase mindestens einen Aminosäure-Austausch aufweist, der dazu führt, dass die diesen mindestens einen Aminosäure-Austausch aufweisende Borneol-Dehydrogenase enantioselektiv Campher umsetzt.

Die erfindungsgemäß eingesetzte enantioselektive Borneol-Dehydrogenase, insbesondere (+)-Borneol-Dehydrogenase, zeichnet sich in bevorzugter Ausführungsform insbesondere dadurch aus, dass sie eine Aminosäuresequenz aufweist, die der Wildtyp-Aminosäuresequenz der aus Pseudomonas putida (ATCC17453) gleicht, das heißt identisch ist, bis auf mindestens einen Aminosäure-Austausch in dieser Aminosäuresequenz und zwar derartig, dass diese Aminosäuresequenz der erfindungsgemäßen Borneol-Dehydrogenase, insbesondere (+)-Borneol-Dehydrogenase, gegenüber der Aminosäuresequenz der aus Pseudomonas putida (ATCC17453) stammenden Wildtyp-Borneol-Dehydrogenase mindestens einen Aminosäure-Austausch aufweist, der dazu führt, dass die diesen mindestens einen Aminosäure-Austausch aufweisende Borneol-Dehydrogenase, insbesondere (+)-Borneol-Dehydrogenase, enantioselektiv Campher umsetzt.

Der erfindungsgemäß vorgesehene mindestens eine Aminosäure-Austausch ist spezifisch so ausgelegt, dass eine Enantioselektivität des diesen mindestens einen Aminosäure-Austausch aufweisenden Enzyms sichergestellt ist. Der erfindungsgemäß vorgesehene mindestens eine Aminosäure-Austausch ist ein Aminosäure-Austausch, der die Funktionalität der ausgetauschten Aminosäure ändert, das heißt die mindestens eine ausgetauschte Aminosäure der Wildtyp-Aminosäuresequenz mit einer bestimmten Funktionalität wird ersetzt durch eine Aminosäure mit einer anderen Funktionalität. Im Zusammenhang mit der vorliegenden Erfindung werden die natürlicherweise vorkommenden Aminosäuren in fünf Funktionalitätsgruppen unterteilt, nämlich in polare, unpolare, phenolische, saure und basische Funktionalitäten aufweisende Aminosäuren. Demgemäß weist die enantioselektive Borneol-Dehydrogenase, insbesondere (+)-Borneol-Dehydrogenase, in bevorzugter Ausführungsform mindestens einen Aminosäure-Austausch mindestens einer Aminosäure mit einer der fünf Funktionalitäten polar, unpolar, phenolisch, sauer oder basisch auf, die in der Wildtyp-Borneol-Dehydrogenase pflanzlichen oder mikrobiellen Ursprungs vorkommt, insbesondere die in der Wildtyp-Borneol-Dehydrogenase gemäß SEQ ID No. 1 vorkommt, wobei die ausgetauschte Aminosäure durch eine Aminosäure mit einer der vier anderen Funktionalitäten ersetzt wird. Die Erfindung sieht bevorzugt vor, dass mindestens eine der in einer Wildtyp-Aminosäuresequenz, insbesondere in der SEQ ID No. 1, vorhandenen Aminosäuren, die eine der fünf verschiedenen Funktionalitäten aufweist, ausgetauscht wird durch eine Aminosäure, die eine der vier anderen Funktionalitäten aufweist.

Der erfindungsgemäß vorgesehene, mindestens eine Aminosäure-Austausch betrifft in besonders bevorzugter Ausführungsform die Aminosäuren, die die Substratbindestelle der Wildtyp-Borneol-Dehydrogenase formieren, insbesondere der Wildtyp-Borneol-Dehydrogenase gemäß SEQ ID No. 1, insbesondere in einem Bereich von Q96 bis Y188, insbesondere die Aminosäuren aus der Gruppe bestehend aus I18, V92, G94, V95, Q96, R97, H98, S99, L112, N116, F142, G143, S144, E145, S146, G147, L148, T149, G150, E151, I152, D153, N154, G155, L156, Y157, A158, A159, T160, K161, A162, H165, V185, L186, P187, Y188, M189, V190, T191, L201, S202, P203, A205, L206, A207, K214, D216, I217, F223 und L230 (jeweils gemäß SEQ ID No. 1). Diese Aminosäuren, an den angegebenen Positionen, definieren insbesondere die Substratbindestelle der Borneol-Dehydrogenase.

Die erfindungsgemäß bereitgestellte enantioselektive Borneol-Dehydrogenase, insbesondere (+)-Borneol-Dehydrogenase, ist in bevorzugter Ausführungsform eine 1R-(+)-Campher Enantiomeren-selektive Borneol-Dehydrogenase (auch als (+)-Borneol-Dehydrogenase bezeichnet), die enantioselektiv, bevorzugt allein, 1R-(+)-Campher-Enantiomer zu (+)-Borneol umsetzt, sodass das zu erhaltene gewünschte Enantiomer ein 1S-(-)-Campher-Enantiomer ist. In einer weiteren bevorzugten Ausführungsform der vorliegenden Erfindung ist die enantioselektive Borneol-Dehydrogenase eine 1S-(-)-Campher Enantiomeren-selektive Borneol-Dehydrogenase, die enantioselektiv, bevorzugt allein, 1S-(-)-Campher-Enantiomer zu (-)-Borneol umsetzt, sodass das zu erhaltene gewünschte Enantiomer ein 1R-(+)-Campher-Enantiomer ist.

Die Erfindung sieht in einer besonders bevorzugten Ausführungsform vor, dass das in Verfahrensschritt a) bereitgestellte Ausgangsmaterial Campher racemisch oder ein einen Enantiomeren-Überschuss von 1R-(+)-Campher oder 1S-(-)-Campher aufweisendes Gemisch ist. Demgemäß kann in einer Ausführungsform das in Verfahrensschritt a) bereitgestellte Ausgangsmaterial Campher ein 1:1-Verhältnis von 1R-(+)-Campher und 1S-(-)-Campher aufweisen. In einer weiteren Ausführungsform kann das in Verfahrensschritt a) bereitgestellte Ausgangsmaterial Campher ein Gemisch sein, das ein von dem 1:1-Verhältnis von 1S-(-)-Campher zu 1R-(+)-Campher abweichendes Verhältnis der beiden Enantiomere aufweist und demgemäß ein einen Enantiomeren-Überschuss von 1R-(+)-Campher oder 1S-(-)-Campher aufweisendes Gemisch darstellt.

Die vorliegende Erfindung ermöglicht es, sowohl aus dem racemischen Campher als auch aus dem einen Enantiomeren-Überschuss von 1R-(+)-Campher oder 1S-(-)-Campher aufweisenden Gemisch durch die enantioselektive Borneol-Dehydrogenase, insbesondere (+)-Borneol-Dehydrogenase, eine Campher-Enantiomeren-angereicherte Zusammensetzung herzustellen, wobei sich die 1R-(+)-Campher- oder 1S-(-)-Campher-Enantiomeren-angereicherte Zusammensetzung, also das Produkt der erfindungsgemäß ermöglichten Umsetzung, durch ein Verhältnis von 1R-(+)-Campher zu 1S-(-)-Campher Enantiomer auszeichnet, das von dem in dem verwendeten Ausgangsmaterial, also racemischen Campher oder dem einen Enantiomeren-Überschuss von 1R-(+)-Campher- oder 1S-(-)-Campher aufweisenden Gemisch, abweicht. Die erfindungsgemäß vorgesehene Verfahrensweise, in Verfahrensschritt b) eine enantioselektive Borneol-Dehydrogenase, insbesondere (+)-Borneol-Dehydrogenase, einzusetzen, führt dazu, dass sich das Verhältnis der Stoffmengen der beiden im eingesetzten Campher vorhandenen Enantiomere selektiv und gezielt ändert. Demgemäß kann in vorteilhafter Weise einerseits aus einem racemischen Campher-Gemisch eine Campher-Enantiomeren-angereicherte Zusammensetzung hergestellt werden. Andererseits ist es auch möglich, aus einem bereits einen Enantiomeren-Überschuss aufweisenden Gemisch von 1R-(+)-Campher oder 1S-(-)-Campher eine Campher-Enantiomeren-angereicherte Zusammensetzung bereitzustellen, die ein anderes Stoffmengenverhältnis von 1R-(+)-Campher zu 1S-(-)-Campher im Vergleich zu dem in Verfahrens schritt a) eingesetzten, einen Enantiomeren-Überschuss von 1R-(+)-Campher- oder 1S-(-)-Campher-aufweisenden Gemisch aufweist.

Bevorzugt ist erfindungsgemäß vorgesehen, dass der in Verfahrensschritt a) bereitgestellte Campher racemisch ist.

Vorzugsweise ist erfindungsgemäß vorgesehen, dass der in Verfahrensschritt a) bereitgestellte Campher ein einen Enantiomeren-Überschuss von 1R-(+)-Campher oder 1S-(-)-Campher aufweisendes Gemisch ist.

In einer bevorzugten Ausführungsform setzt die in Verfahrensschritt a) bereitgestellte enantioselektive Borneol-Dehydrogenase, insbesondere (+)-Borneol-Dehydrogenase, enantioselektiv 1R-(+)-Campher oder 1S-(-)-Campher um.

In besonders bevorzugter Ausführungsform ist die in Verfahrensschritt a) bereitgestellte enantioselektive Borneol-Dehydrogenase eine enantioselektive (+)-Borneol-Dehydrogenase, die in der Lage ist, enantioselektiv 1R(+)-Campher umzusetzen, insbesondere so, dass eine 1S-(-)-Campher-Enantiomeren-angereicherte Zusammensetzung erhalten wird. In besonders bevorzugter Ausführungsform ist die in Verfahrensschritt a) bereitgestellte enantioselektive Borneol-Dehydrogenase eine enantioselektive (-)-Borneol-Dehydrogenase, die in der Lage ist, enantioselektiv 1S-(-)-Campher umzusetzen, insbesondere so, dass in Verfahrensschritt c) eine 1R-(+)-Campher-Enantiomeren-angereicherte Zusammensetzung erhalten wird.

Bevorzugt ist erfindungsgemäß vorgesehen, dass die in Verfahrensschritt a) bereitgestellte enantioselektive Borneol-Dehydrogenase, insbesondere (+)-Borneol-Dehydrogenase, pflanzlichen oder mikrobiellen Ursprungs, bevorzugt mikrobiellen Ursprungs, oder synthetisch hergestellt ist.

In einer besonders bevorzugten Ausführungsform kann in Verfahrensschritt a) neben der bereitgestellten enantioselektiven Borneol-Dehydrogenase, insbesondere (+)-Borneol-Dehydrogenase" dem Reaktionsmedium und dem Campher optional mindestens ein Puffersystem, optional mindestens ein reduzierender Co-Faktor (erstes Substrat) sowie optional mindestens ein Regenerationssystem bereitgestellt werden. Besonders bevorzugt wird insbesondere Campher, ein Reaktionsmedium, eine enantioselektive Borneol-Dehydrogenase, insbesondere (+)-Borneol-Dehydrogenase,, ein Puffersystem, ein reduzierender Co-Faktor und ein Regenerationssystem bereitgestellt.

In einer besonders bevorzugten Ausführungsform ist das in Verfahrensschritt a) bereitgestellte Reaktionsmedium eine wässrige Lösung, insbesondere eine wässrige gepufferte Lösung, insbesondere wird Verfahrensschritt b) in einer wässrigen gepufferten Lösung durchgeführt. Eine gepufferte Lösung ist im Zusammenhang mit der vorliegenden Erfindung eine Lösung enthaltend ein Puffersystem.

In besonders bevorzugter Ausführungsform ist vorgesehen, dass Verfahrensschritt b) in Anwesenheit mindestens eines Puffersystems zum Beispiel eines Citrat-Phosphat-Puffers, eines Citrat-Puffers, eines Phosphat-Puffers oder eines Essigsäure-Acetat-Puffers durchgeführt wird.

In einer bevorzugten Ausführungsform weist das Reaktionsmedium in Verfahrensschritt b) eine Temperatur von 20 °C bis 45 °C, insbesondere 25 °C bis 40 °C, insbesondere 28 °C bis 32 °C, insbesondere 30 °C auf.

In einer bevorzugten Ausführungsform weist das Reaktionsmedium in Verfahrensschritt b) einen pH-Wert von pH 2,6 bis pH 8,0, insbesondere pH 3,7 bis pH 6,2, insbesondere pH 4,0 bis pH 6,5, insbesondere pH 4,0 bis pH 6,0, insbesondere pH 4,0 bis pH 5,0, insbesondere pH 4,5 auf. Bevorzugt liegt der pH-Wert bei pH 4,0 bis pH 6,5.

In einer besonderes bevorzugten Ausführungsform weist das Reaktionsmedium in Verfahrensschritt b) eine Temperatur und einen pH-Wert von 20 °C bis 45 °C und pH 4,0 bis pH 6,5, insbesondere 25 °C bis 40 °C und pH 4,0 bis pH 6,0, insbesondere 28 °C bis 32 °C und pH 4,0 bis pH 5,0, insbesondere 30 °C und pH 4,5 auf.

Bevorzugt ist erfindungsgemäß vorgesehen, dass das Umsetzen in Verfahrensschritt b), insbesondere die Reduktion, unter Anwesenheit mindestens eines reduzierenden Co-Faktors in dem Reaktionsmedium durchgeführt wird, insbesondere NADH oder NADPH, bevorzugt NADH, oder einem NADH/NADPH-Analogon.

In einer besonders bevorzugten Ausführungsform findet das Umsetzen in Verfahrensschritt b) in Anwesenheit eines Regenerationssystems statt. Dieses Regenerationssystem dient einer Regeneration des bevorzugt eingesetzten reduzierenden Co-Faktors. In bevorzugter Ausführungsform dient das Regenerationssystem der Reduktion des bevorzugt für die Campher-Umsetzung eingesetzten und dabei oxidierten reduzierenden Co-Faktors.

In einer besonders bevorzugten Ausführungsform weist das Reaktionsmedium in Verfahrensschritt b) einen reduzierenden Co-Faktor, insbesondere mit oder ohne Regenerationssystem, insbesondere 1 bis 4000 mol-%, insbesondere 1 bis 3500 mol-%, insbesondere 1 bis 3000 mol-%, insbesondere 1 bis 2500 mol-%, insbesondere 1 bis 2000 mol-% (bezogen auf den eingesetzten Campher) des reduzierenden Co-Faktors auf.

In einer bevorzugten Ausführungsform weist das Reaktionsmedium in Verfahrensschritt b) einen reduzierenden Co-Faktor ohne Regenerationssystem, insbesondere 80 bis 4000 mol-%, insbesondere 80 bis 3500 mol-%, insbesondere 80 bis 3000 mol-%, insbesondere 80 bis 2500 mol-%, insbesondere 80 bis 2000 mol-% (bezogen auf den eingesetzten Campher) des reduzierenden Co-Faktors auf.

In einer bevorzugten Ausführungsform weist das Reaktionsmedium in Verfahrensschritt b) mindestens einen reduzierenden Co-Faktor und mindestens ein Regenerationssystem, insbesondere einen reduzierenden Co-Faktor und ein Regenerationssystem auf, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors auf.

In einer bevorzugten Ausführungsform weist das Reaktionsmedium in Verfahrensschritt b) mindestens einen reduzierenden Co-Faktor und mindestens ein Regenerationssystem auf, wobei das Regenerationssystem ein elektrochemisches Regenerationssystem oder ein enzymatisches Regenerationssystem ist.

In einer bevorzugten Ausführungsform weist das enzymatische Regenerationssystem ein zur Reduktion befähigtes Enzym auf, insbesondere ein zur Reduktion eines zweiten Substrats befähigtes Enzym, insbesondere eine Dehydrogenase, insbesondere eine Glycosyl- oder Formiat-Dehydrogenase, insbesondere eine Glucose- oder Formiat-Dehydrogenase und ein zweites Substrat, insbesondere ein von dem zur Reduktion befähigten Enzym zu reduzierendes zweites Substrat. In bevorzugter Ausführungsform ist das zweite Substrat insbesondere ein Zucker, insbesondere ein Einfachzucker, insbesondere Glucose oder eine Säure, insbesondere Ameisensäure.

In einer bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C und einen reduzierenden Co-Faktor mit oder ohne Regenerationssystem insbesondere 1 bis 4000 mol-%, insbesondere 1 bis 3500 mol-%, insbesondere 1 bis 3000 mol-%, insbesondere 1 bis 2500 mol-%, insbesondere 1 bis 2000 mol-% (bezogen auf den eingesetzten Campher) des reduzierenden Co-Faktors aufweist.

In einer besonders bevorzugen Ausführungsform ist erfindungsgemäß vorgesehen, dass das Reaktionsmedium in Verfahrensschritt b) einen pH-Wert von pH 2,6 bis pH 8,0, insbesondere 4,0 bis pH 6,5, insbesondere pH 4,0 bis pH 5,0, insbesondere pH 4,5 und einen reduzierenden Co-Faktor, insbesondere einen reduzierenden Co-Faktor mit oder ohne Regenerationssystem, insbesondere 1 bis 4000 mol-%, insbesondere 1 bis 3500 mol-%, insbesondere 1 bis 3000 mol-%, insbesondere 1 bis 2500 mol-%, insbesondere 1 bis 2000 mol-% (bezogen auf den eingesetzten Campher) des reduzierenden Co-Faktors aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 4,0 bis pH 6,5, insbesondere pH 4,0 bis pH 6,0, insbesondere pH 4,0 bis pH 5,0, insbesondere pH 4,5 und einen reduzierenden Co-Faktor, insbesondere einen reduzierenden Co-Faktor mit oder ohne Regenerationssystem, insbesondere 1 bis 4000 mol-%, insbesondere 1 bis 3500 mol-%, insbesondere 1 bis 3000 mol-%, insbesondere 1 bis 2500 mol-%, insbesondere 1 bis 2000 mol-% (bezogen auf den eingesetzten Campher) des reduzierenden Co-Faktors aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor mit oder ohne Regenerationssystem, insbesondere 1 bis 4000 mol-%, insbesondere 1 bis 3500 mol-%, insbesondere 1 bis 3000 mol-%, insbesondere 1 bis 2500 mol-%, insbesondere 1 bis 2000 mol-% (bezogen auf den eingesetzten Campher) des reduzierenden Co-Faktors und mindestens ein Puffersystem aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors und als zweites Substrat einen Zucker, insbesondere einen Einfachzucker, insbesondere Glucose aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors, ein Puffersystem und als zweites Substrat einen Zucker, insbesondere einen Einfachzucker, insbesondere Glucose aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors und als zur Reduktion befähigtes Enzym, insbesondere als zur Reduktion des zweiten Substrats befähigtes Enzym, insbesondere eine Glucose-Dehydrogenase, insbesondere wenn in dem Reaktionsmedium als zweites Substrat ein Zucker, insbesondere Glucose, vorliegt, aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors, ein Puffersystem, als zur Reduktion befähigtes Enzym, insbesondere als zur Reduktion des zweiten Substrats befähigtes Enzym, insbesondere eine Glucose-Dehydrogenase, insbesondere wenn in dem Reaktionsmedium als zweites Substrat ein Zucker, insbesondere Glucose, vorliegt, aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors und als zweites Substrat eine Säure, insbesondere Ameisensäure, aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors, ein Puffersystem und als zweites Substrat eine Säure, insbesondere Ameisensäure, aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors und als zur Reduktion befähigtes Enzym, insbesondere als zur Reduktion des zweiten Substrats befähigtes Enzym, insbesondere eine Formiat-Dehydrogenase, insbesondere wenn in dem Reaktionsmedium als zweites Substrat eine Säure, insbesondere Ameisensäure, vorliegt, aufweist.

In einer besonders bevorzugten Ausführungsform ist erfindungsgemäß vorgesehen, dass das Reaktionsmedium in Verfahrensschritt b) eine Temperatur von 20 °C bis 45 °C, insbesondere 25 °C bis 40 °C, insbesondere 28 °C bis 32 °C, insbesondere 30 °C, einen pH-Wert von pH 2,6 bis pH 8,0, insbesondere pH 4,0 bis pH 6,5, insbesondere pH 4,0 bis pH 5,0, insbesondere pH 4,5, einen reduzierenden Co-Faktor, insbesondere einen reduzierenden Co-Faktor und ein Regenerationssystem, insbesondere 1 bis 2000 mol-%, insbesondere 1 bis 1800 mol-%, insbesondere 1 bis 1500 mol-%, insbesondere 1 bis 1200 mol-%, insbesondere 1 bis 1000 mol-% (bezogen auf den eingesetzten Campher) des reduzierenden Co-Faktors, ein Puffersystem und als zur Reduktion befähigtes Enzym, insbesondere als zur Reduktion des zweiten Substrats befähigtes Enzym, insbesondere eine Formiat-Dehydrogenase, insbesondere wenn in dem Reaktionsmedium als zweites Substrat eine Säure, insbesondere Ameisensäure, vorliegt, aufweist.

In besonders bevorzugter Ausführungsform wird in Verfahrensschritt b) der in Verfahrensschritt a) bereitgestellte Campher, insbesondere racemischer Campher oder ein einen Enantiomeren-Überschuss von 1S-(-)-Campher oder 1R-(+)-Campher aufweisendes Gemisch so umgesetzt, dass dadurch die in Verfahrensschritt c) erhaltene 1R-(+)-Campher- oder 1S(-)-Campher-Enantiomeren-angereicherte Zusammensetzung einen um mindestens 1, mindestens 10, mindestens 20, mindestens 40, mindestens 60, mindestens 80, mindestens 100 % (bezogen auf den Anteil des im Ausgangsmaterial vorliegenden 1R-(+)-Campher) reduzierten Anteil von 1R-(+)-Campher aufweist.

In besonders bevorzugter Ausführungsform wird in Verfahrensschritt b) der in Verfahrensschritt a) bereitgestellte Campher, insbesondere racemischer Campher oder ein einen Enantiomeren-Überschuss von 1S-(-)-Campher oder 1R-(+)-Campher aufweisendes Gemisch so umgesetzt, dass dadurch die in Verfahrensschritt c) erhaltene 1R-(+)-Campher- oder 1S(-)-Campher-Enantiomeren-angereicherte Zusammensetzung einen um mindestens 1, mindestens 10, mindestens 20, mindestens 40, mindestens 60, mindestens 80, mindestens 100 % (bezogen auf den Anteil des im Ausgangsmaterial vorliegenden 1S-(-)-Campher) reduzierten Anteil von 1S-(-)-Campher aufweist.

In einer bevorzugten Ausführungsform wird das in Verfahrensschritt c) erhaltene Gemisch aus 1R-(+)-Campher und (-)-Borneol oder aus (1S)-(-)-Campher und (+)-Borneol in einem Verfahrensschritt d) mittels selektiver chemischer Umsetzung oder einer physikalischen Auftrennmethode in mindestens eine Campher- und eine Borneol-haltige Fraktion aufgetrennt, insbesondere mit mindestens einer Chromatographie- oder Destillationsmethode oder Kristallisation.

In einer besonders bevorzugten Ausführungsform wird das in Verfahrensschritt d) erhaltene (+)-Borneol oder (-)-Borneol in einem Verfahrensschritt e) zu 1R-(+)-Campher oder (1S)-(-)-Campher umgesetzt.

In einer besonders bevorzugten Ausführungsform wird die aus den Verfahrensschritten a) bis c) erhaltene 1R-(+)-Campher-Enantiomeren-angereicherte Zusammensetzung oder (1S)-(-)-Campher-Enantiomeren-angereicherte Zusammensetzung in einem Verfahrensschritt f) zu 1R-(-)-Campher-10-Sulfonsäure oder 1S-(+)-Campher-10-Sulfonsäure umgesetzt, wobei der Verfahrensschritt f) folgende Schritte f1) bis f4) umfasst:

f1) Zugeben von Schwefelsäure zu der 1R-(+)-Campher-Enantiomeren-angereicherten oder (1S)-(-)-Campher-Enantiomeren-angereicherten Zusammensetzung in einem organischen Lösungsmittel, insbesondere Acetanhydrid,
f2) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
f3) Erhalten einer Zusammensetzung von 1R-(-)-Campher-10-Sulfonsäure und (- )-Borneol oder 1S-(+)-Campher-10-Sulfonsäure und (+)-Borneol, wobei vorzugsweise anschließend erfolgt ein
f4) Auftrennen der erhaltenen Zusammensetzung von 1R-(-)-Campher-10-Sulfonsäure und (-)-Borneol oder 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

In einer besonders bevorzugten Ausführungsform wird das in Verfahrensschritt f4) erhaltene (+)-Borneol oder (-)-Borneol in einem Verfahrensschritt g) zu 1R-(+)-Campher oder (1S)-(-)-Campher umgesetzt.

In einer besonders bevorzugten Ausführungsform wird das aus den Verfahrensschritten d), e), g) erhaltene 1R-(+)-Campher oder 1S-(-)-Campher in einem Verfahrensschritt h) zu 1R-(-)-Campher-10-Sulfonsäure oder 1S-(+)-Campher-10-Sulfonsäure umgesetzt, wobei der Verfahrensschritt h) folgende Schritte h1) bis h3) umfasst:

h1) Zugeben von Schwefelsäure zu 1R-(+)-Campher oder (1S)-(-)-Campher in einem organischen Lösungsmittel, insbesondere Acetanhydrid,
h2) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
h3) Erhalten von 1R-(-)-Campher-10-Sulfonsäure oder 1S-(+)-Campher-10-Sulfonsäure.

In einer besonders bevorzugter Ausführungsform ist vorgesehen, dass in Verfahrensschritt f1) oder h1) Schwefelsäure, insbesondere konzentrierte Schwefelsäure, insbesondere 100 bis 1000 mol-%, insbesondere 100 bis 800 mol-%, insbesondere 100 bis 500 mol-%, insbesondere 100 bis 300 mol-%, vorzugsweise 100 mol-% (bezogen auf den eingesetzten Campher) Schwefelsäure zugegeben wird.

In einer besonders bevorzugten Ausführungsform ist vorgesehen, dass in Verfahrensschritt f1) oder h1) ein organisches Lösungsmittel, insbesondere Acetanhydrid, insbesondere 200 bis 2000 mol-%, insbesondere 200 bis 1600 mol-%, insbesondere 200 bis 1000 mol-%, insbesondere 200 bis 600 mol-%, vorzugsweise 200 mol-% (bezogen auf den eingesetzten Campher) eines organischen Lösungsmittels, insbesondere Acetanhydrid, eingesetzt wird.

In einer besonders bevorzugten Ausführungsform ist vorgesehen, dass in Verfahrensschritt f1) oder h1) Schwefelsäure, insbesondere konzentrierte Schwefelsäure, insbesondere 100 bis 1000 mol-%, insbesondere 100 bis 800 mol-%, insbesondere 100 bis 500 mol-%, insbesondere 100 bis 300 mol-%, vorzugsweise 100 mol-% (bezogen auf den eingesetzten Campher) Schwefelsäure, und ein organisches Lösungsmittel, insbesondere Acetanhydrid, insbesondere 200 bis 2000 mol-%, insbesondere 200 bis 1600 mol-%, insbesondere 200 bis 1000 mol-%, insbesondere 200 bis 600 mol-%, vorzugsweise 200 mol-% (bezogen auf den eingesetzten Campher) eines organischen Lösungsmittels, insbesondere Acetanhydrid, eingesetzt werden.

In besonders bevorzugter Ausführungsform wird in Verfahrensschritt f1) oder h1) die Schwefelsäure, insbesondere konzentrierte Schwefelsäure, das organische Lösungsmittel, insbesondere Acetanhydrid und der eingesetzte Campher in einem besonders bevorzugten Stoffmengenverhältnis von 1:2:1 eingesetzt.

Erfindungsgemäß betrifft die vorliegende Erfindung auch ein Verfahren zur Herstellung von 1R-(-)-Campher-10-Sulfonsäure oder 1S-(+)-Campher-10-Sulfonsäure umfassend die folgenden Verfahrensschritte i1) bis i5):

i1) Durchführen der Verfahrensschritte a) bis c),
i2) Zugeben von Schwefelsäure zu der 1R-(+)-Campher-Enantiomeren-angereicherten oder (1S)-(-)-Campher-Enantiomeren-angereicherten Zusammensetzung in einem organischen Lösungsmittel, insbesondere Acetanhydrid,
i3) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
i4) Erhalten einer Zusammensetzung von 1R-(-)-Campher-10-Sulfonsäure und (-)-Borneol oder 1S-(+)-Campher-10-Sulfonsäure und (+)-Borneol, wobei vorzugsweise anschließend erfolgt ein
i5) Auftrennen der erhaltenen Zusammensetzung von 1R-(-)-Campher- 10-Sulfonsäure und (-)-Borneol oder 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

In einer bevorzugten Ausführungsform wird das aus Verfahrensschritt i5) erhaltene (+)-Borneol oder (-)-Borneol in einem Verfahrensschritt j) zu 1R-(+)-Campher oder 1S-(-)-Campher umgesetzt.

Erfindungsgemäß betrifft die vorliegende Erfindung auch ein Verfahren zur Herstellung von 1R-(-)-Campher-10-Sulfonsäure oder 1S-(+)-Campher-10-Sulfonsäure umfassend die folgenden Verfahrens schritte k1) bis k4):

k1) Durchführen der Verfahrensschritte nach Verfahrensschritte a) bis d), oder Verfahrensschritte a) bis e), oder Verfahrensschritte a) bis g), oder Verfahrensschritte a) bis j),
k2) Zugeben von Schwefelsäure zu dem aus Verfahrensschritt d), e), g) oder j) erhaltenen 1R-(+)-Campher oder (1S)-(-)-Campher in einem organischen Lösungsmittel, insbesondere Acetanhydrid,
k3) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
k4) Erhalten von 1R-(-)-Campher-10-Sulfonsäure oder 1S-(+)-Campher-10-Sulfonsäure.

In einer besonders bevorzugter Ausführungsform ist vorgesehen, dass in Verfahrensschritt i2) oder k2) Schwefelsäure, insbesondere konzentrierte Schwefelsäure, insbesondere 100 bis 1000 mol-%, insbesondere 100 bis 800 mol-%, insbesondere 100 bis 500 mol-%, insbesondere 100 bis 300 mol-%, vorzugsweise 100 mol-% (bezogen auf den eingesetzten Campher) Schwefelsäure zugegeben wird.

In einer besonders bevorzugten Ausführungsform ist vorgesehen, dass in Verfahrensschritt i2) oder k2) ein organisches Lösungsmittel, insbesondere Acetanhydrid, insbesondere 200 bis 2000 mol-%, insbesondere 200 bis 1600 mol-%, insbesondere 200 bis 1000 mol-%, insbesondere 200 bis 600 mol-%, vorzugsweise 200 mol-% (bezogen auf den eingesetzten Campher) eines organischen Lösungsmittels, insbesondere Acetanhydrid, eingesetzt wird.

In einer besonders bevorzugten Ausführungsform ist vorgesehen, dass in Verfahrensschritt i2) oder k2) Schwefelsäure, insbesondere konzentrierte Schwefelsäure, insbesondere 100 bis 1000 mol-%, insbesondere 100 bis 800 mol-%, insbesondere 100 bis 500 mol-%, insbesondere 100 bis 300 mol-%, vorzugsweise 100 mol-% (bezogen auf den eingesetzten Campher) Schwefelsäure, und ein organisches Lösungsmittel, insbesondere Acetanhydrid, insbesondere 200 bis 2000 mol-%, insbesondere 200 bis 1600 mol-%, insbesondere 200 bis 1000 mol-%, insbesondere 200 bis 600 mol-%, vorzugsweise 200 mol-% (bezogen auf den eingesetzten Campher) eines organischen Lösungsmittels, insbesondere Acetanhydrid, eingesetzt werden.

In besonders bevorzugter Ausführungsform wird in Verfahrensschritt i2) oder k2) die Schwefelsäure, insbesondere konzentrierte Schwefelsäure, das organische Lösungsmittel, insbesondere Acetanhydrid und der eingesetzte Campher in einem besonders bevorzugten Stoffmengenverhältnis von 1:2:1 eingesetzt.

Erfindungsgemäß betrifft die vorliegende Erfindung auch eine enantioselektive Borneol-Dehydrogenase, insbesondere (+)-Borneol-Dehydrogenase, umfassend mindestens einen Aminosäure-Austausch mindestens einer Aminosäure mit einer der fünf Funktionalitäten polar, unpolar, phenolisch, sauer oder basisch einer Wildtyp-Borneol-Dehydrogenase, insbesondere der Wildtyp-Borneol-Dehydrogenase gemäß SEQ ID No. 1, dadurch gekennzeichnet, dass die ausgetauschte Aminosäure durch eine Aminosäure mit einer der vier anderen Funktionalitäten ersetzt wird.

Die erfindungsgemäß bereitgestellte enantioselektive Borneol-Dehydrogenase, insbesondere (+)-Borneol-Dehydrogenase, weist daher die Aminosäuresequenz einer Wildtyp-Borneol-Dehydrogenase, insbesondere der Wildtyp-Borneol-Dehydrogenase gemäß SEQ ID No. 1 auf, mit der Ausnahme, dass in dieser Wildtyp-Sequenz, insbesondere gemäß der SEQ ID No. 1, mindestens ein Aminosäure-Austausch durchgeführt wurde, sodass die ausgetauschte Aminosäure, welche entweder eine polare, unpolare, phenolische, saure oder basische Aminosäure ist, durch eine Aminosäure ausgetauscht wurde, die eine Funktionalität aufweist, welche von der Funktionalität der im Wildtyp vorliegenden Aminosäure abweicht. Dieser mindestens eine Aminosäure-Austausch führt dazu, dass die Wildtyp-Borneol-Dehydrogenase ihre nicht vorhandene Enantioselektivität verliert und enantioselektiv wird.

In einer bevorzugten Ausführungsform weist die erfindungsgemäße enantioselektive Borneol-Dehydrogenase, insbesondere (+)-Borneol-Dehydrogenase, einen Aminosäure-Austausch mindestens einer Aminosäure mit einer der fünf Funktionalitäten polar, unpolar, phenolisch, sauer oder basisch der Wildtyp-Borneol-Dehydrogenase, insbesondere der Wildtyp-Borneol-Dehydrogenase gemäß SEQ ID No. 1 auf, dadurch gekennzeichnet, dass die ausgetauschte Aminosäure durch eine Aminosäure mit einer der vier anderen Funktionalitäten ersetzt wird, insbesondere mindestens einer Aminosäure mit einer polaren und/oder unpolaren Funktionalität.

In bevorzugter Ausführungsform betrifft die vorliegende Erfindung eine enantioselektive Borneol-Dehydrogenase, insbesondere (+)-Borneol-Dehydrogenase, wobei die mindestens eine in der SEQ ID No. 1 ausgetauschte Aminosäure ausgewählt ist aus der Gruppe bestehend aus I18, V92, G94, V95, Q96, R97, H98, S99, L112, N116, F142, G143, S144, E145, S146, G147, L148, T149, G150, E151, I152, D153, N154, G155, L156, Y157, A158, A159, T160, K161, A162, H165, V185, L186, P187, Y188, M189, V190, T191, L201, S202, P203, A205, L206, A207, K214, D216, I217, F223 und L230 (jeweils gemäß SEQ ID No. 1). Die Aminosäuren an den angegebenen Positionen definieren insbesondere die Substratbindestelle der Borneol-Dehydrogenase.

In bevorzugter Ausführungsform betrifft die vorliegende Erfindung eine enantioselektive Borneol-Dehydrogenase, insbesondere (+)-Borneol-Dehydrogenase, wobei die mindestens eine in der SEQ ID No. 1 ausgetauschte Aminosäure ausgewählt ist aus der Gruppe bestehend aus I18, V95, Q96, R97, H98, S144, E145, S146, E151, I152,D153, N154, Y157, A158, K161, P187, Y188, M189, T191 und S202 (jeweils gemäß SEQ ID No. 1).

In bevorzugter Ausführungsform betrifft die vorliegende Erfindung eine enantioselektive Borneol-Dehydrogenase, insbesondere (+)-Borneol-Dehydrogenase, wobei die mindestens eine in der SEQ ID No. 1 ausgetauschte Aminosäure ausgewählt ist aus der Gruppe in einem Bereich von Q96 bis Y188, insbesondere ausgewählt ist aus der Gruppe bestehend aus Q96, H98, E145, S146, N154 und Y188 (jeweils gemäß SEQ ID No. 1).

In bevorzugter Ausführungsform betrifft die vorliegende Erfindung eine enantioselektive Borneol-Dehydrogenase, insbesondere (+)-Borneol-Dehydrogenase, wobei die mindestens eine in der SEQ ID No. 1 ausgetauschte Aminosäure ausgewählt ist aus der Gruppe bestehend aus Q96, H98, E145, S146, N154 und Y188 (jeweils gemäß SEQ ID No. 1) und wobei die Aminosäure Q96 ausgetauscht ist durch R, F, Y, W, K, E, N, L, M, H98 ausgetauscht ist durch F, Y, B, Q, R, K, E, L, M, N, E145 ausgetauscht ist durch M, L, N, I, S, H, K, R, Q, S, Y, W, S146 ausgetauscht ist durch G, A, V, I, L, N, D, E, T, N154 ausgetauscht ist durch L, E, M, E, S, A, K, W, Y, F, Q, R und/oder Y188 ausgetauscht ist durch W, H, R, Q, F, K, E, L, M, N.

In bevorzugter Ausführungsform betrifft die vorliegende Erfindung eine enantioselektive Borneol-Dehydrogenase, insbesondere (+)-Borneol-Dehydrogenase, wobei diese einen oder zwei Aminosäure-Austausche aufweist, wobei wenn ein Aminosäure-Austausch vorliegt, dieser insbesondere an einer der Positionen E145, S146 oder Y188 erfolgt, oder wobei wenn zwei Aminosäure-Austausche vorliegen, diese an Positionen S146 und Y188 oder E145 und Y188 erfolgen (jeweils gemäß SEQ ID No. 1).

In bevorzugter Ausführungsform betrifft die vorliegende Erfindung eine enantioselektive Borneol-Dehydrogenase, insbesondere (+)-Borneol-Dehydrogenase, wobei die Aminosäure E145 ausgetauscht ist durch H, M, L, S oder G (jeweils gemäß SEQ ID No. 1).

In bevorzugter Ausführungsform betrifft die vorliegende Erfindung eine enantioselektive Borneol-Dehydrogenase, insbesondere (+)-Borneol-Dehydrogenase, wobei die Aminosäure Y188 ausgetauscht ist durch T, A, V, I oder L (jeweils gemäß SEQ ID No. 1).

In bevorzugter Ausführungsform betrifft die vorliegende Erfindung eine enantioselektive Borneol-Dehydrogenase, insbesondere (+)-Borneol-Dehydrogenase, wobei die Aminosäure S146 ausgetauscht ist durch A (SEQ ID No. 3).

In einer bevorzugten Ausführungsform weist die erfindungsgemäße enantioselektive Borneol-Dehydrogenase, insbesondere (+)-Borneol-Dehydrogenase, einen Aminosäure-Austausch der Aminosäure E145 mit einer Aminosäure ausgewählt aus G (SEQ ID No. 5), H (SEQ ID No. 7), M (SEQ ID No. 9), L (SEQ ID No. 11), S (SEQ ID No. 13) auf, insbesondere S.

In einer besonders bevorzugten Ausführungsform weist die erfindungsgemäße enantioselektive Borneol-Dehydrogenase, insbesondere (+)-Borneol-Dehydrogenase, einen Aminosäure-Austausch der Aminosäure Y188 mit einer Aminosäure ausgewählt aus L (SEQ ID No. 15), I (SEQ ID No. 17), V (SEQ ID No. 19), T (SEQ ID No. 21), A (SEQ ID No. 23) auf, insbesondere A.

In einer besonders bevorzugten Ausführungsform weist die erfindungsgemäße enantioselektive Borneol-Dehydrogenase, insbesondere (+)-Borneol-Dehydrogenase, zwei Aminosäure-Austausche auf, wobei der erste Aminosäure-Austausch ein Aminosäure-Austausch der Aminosäure E145 mit einer Aminosäure ausgewählt aus M, L, S, insbesondere L ist und der zweite Aminosäure-Austausch ein Aminosäure-Austausch der Aminosäure Y188 mit einer Aminosäure ausgewählt aus T, A, insbesondere T ist.

In einer besonders bevorzugten Ausführungsform weist die erfindungsgemäße enantioselektive Borneol-Dehydrogenase, insbesondere (+)-Borneol-Dehydrogenase, zwei Aminosäure-Austausche auf, wobei der erste Aminosäure-Austausch ein Aminosäure-Austausch der Aminosäure S146 mit A ist, und der zweite Aminosäure-Austausch ein Aminosäure-Austausch der Aminosäure Y188 mit einer Aminosäure ausgewählt aus T, A, insbesondere T ist.

In bevorzugter Ausführungsform betrifft die vorliegende Erfindung eine enantioselektiven Borneol-Dehydrogenase, insbesondere (+)-Borneol-Dehydrogenase, wobei die Borneol-Dehydrogenase eine Enantioselektivität von bevorzugt mindestens 10 und/oder einen Umsatz von bevorzugt mindestens 10 % (bezogen auf racemischen Campher) aufweist.

In bevorzugter Ausführungsform betrifft die vorliegende Erfindung eine enantioselektive (+)-Borneol-Dehydrogenase, wobei die erfindungsgemäße enantioselektive (+)-Borneol-Dehydrogenase ein Enantiomeren-Verhältnis (E(+)) von mindestens 6 (E(+)), mindestens 7 (E(+)), mindestens 12 (E(+)), mindestens 30 (E(+)), mindestens 50 (E(+)), mindestens 70 (E(+)), mindestens 90 (E(+)), mindestens 100 (E(+)), mindestens mehr als 100 (E(+)), aufweist.

In bevorzugter Ausführungsform betrifft die vorliegende Erfindung eine enantioselektive (+)-Borneol-Dehydrogenase aufweisend einen Aminosäure-Austausch, wobei die erfindungsgemäße enantioselektive (+)-Borneol-Dehydrogenase aufweisend einen Aminosäure-Austausch ein Enantiomeren-Verhältnis (E(+)) von mindestens 6 (E(+)), mindestens 7 (E(+)), mindestens 10 (E(+)), mindestens 12 (E(+)), mindestens 100 (E(+)), mindestens mehr als 100 (E(+)) aufweist.

In bevorzugter Ausführungsform betrifft die vorliegende Erfindung eine enantioselektive (+)-Borneol-Dehydrogenase aufweisend zwei Aminosäure-Austausche, wobei die erfindungsgemäße enantioselektive (+)-Borneol-Dehydrogenase aufweisend zwei Aminosäure-Austausche ein Enantiomeren-Verhältnis (E(+)) von mindestens 30 (E(+)), mindestens 40 (E(+)), mindestens 60 (E(+)), mindestens 70 (E(+)), mindestens 90 (E(+)), mindestens 100 (E(+)), mindestens mehr als 100 (E(+)) aufweist.

In bevorzugter Ausführungsform betrifft die vorliegende Erfindung eine enantioselektive (+)-Borneol-Dehydrogenase, wobei die erfindungsgemäße enantioselektive (+)-Borneol-Dehydrogenase einen Umsatz von mindestens 2 %, mindestens 10 %, mindestens 20 %, mindestens 30 %, mindestens 40 %, insbesondere 50 % (bezogen auf racemischen Campher) aufweist.

In bevorzugter Ausführungsform betrifft die vorliegende Erfindung eine enantioselektive (+)-Borneol-Dehydrogenase aufweisend einen Aminosäure-Austausch, wobei die erfindungsgemäße enantioselektive (+)-Borneol-Dehydrogenase aufweisend einen Aminosäure-Austausch einen Umsatz von mindestens 5 %, mindestens 10 %, mindestens 20 %, mindestens 30 %, mindestens 40 %, insbesondere 50 % (bezogen auf racemischen Campher) aufweist.

In bevorzugter Ausführungsform betrifft die vorliegende Erfindung eine enantioselektive (+)-Borneol-Dehydrogenase aufweisend zwei Aminosäure-Austausche, wobei die erfindungsgemäße enantioselektive (+)-Borneol-Dehydrogenase aufweisend zwei Aminosäure-Austausche einen Umsatz von mindestens 2 %, mindestens 10 %, mindestens 20 %, mindestens 30 %, mindestens 35 %, insbesondere mindestens 40 % (bezogen auf racemischen Campher) aufweist.

In bevorzugter Ausführungsform betrifft die vorliegende Erfindung eine enantioselektive (+)-Borneol-Dehydrogenase, wobei die erfindungsgemäße enantioselektive (+)-Borneol-Dehydrogenase einen Umsatz und ein Enantiomeren-Verhältnis (E(+)) von mindestens 2 % und 30 (E(+)), 5 % und 6 (E(+)), mindestens 11 % und 6 (E(+)), mindestens 17 % und 12 (E(+)), mindestens 29 % und 6 (E(+)), mindestens 50 % und 7 (E(+)), mindestens 5 % und 50 (E(+)), mindestens 10 % und 72 (E(+)), mindestens 11 % und mehr als 100 (E(+)), mindestens 16 % und mehr als 100 (E(+)), mindestens 22 % und mehr als 100 (E(+)), mindestens 40 % und mehr als 100 (E(+)) (Umsatz bezogen auf racemischen Campher) aufweist.

In bevorzugter Ausführungsform betrifft die vorliegende Erfindung eine enantioselektive (+)-Bomeol-Dehydrogenase aufweisend einen Aminosäure-Austausch, wobei die erfindungsgemäße enantioselektive (+)-Borneol-Dehydrogenase aufweisend einen Aminosäure-Austausch einen Umsatz und ein Enantiomeren-Verhältnis (E(+)) von mindestens 5 % und 6 (E(+)), mindestens 8% und 6 (E(+)), mindestens 11 % und 6 (E(+)), mindestens 29 % und 6 (E(+)), mindestens 50 % und 7 (E(+)), mindestens 17 % und 12 (E(+)), mindestens 22 % und mehr als 100 (E(+)) (+)) (Umsatz bezogen auf racemischen Campher) aufweist.

In bevorzugter Ausführungsform betrifft die vorliegende Erfindung eine enantioselektive (+)-Bomeol-Dehydrogenase aufweisend zwei Aminosäure-Austausche, wobei die erfindungsgemäße enantioselektive (+)-Borneol-Dehydrogenase aufweisend zwei Aminosäure-Austausche einen Umsatz und ein Enantiomeren-Verhältnis (E(+)) von mindestens 2 % und 30 (E(+)), mindestens 5 % und 50 (E(+)), mindestens 10 % und 70 (E(+)), mindestens 16 % und mehr als 100 (E(+)), mindestens 39 % und mehr als 100 (E(+)), mindestens 40 % und mehr als 100 (E(+)) (+)) (Umsatz bezogen auf racemischen Campher) aufweist.

In einer besonders bevorzugten Ausführungsform weist die erfindungsgemäße enantioselektive (+)-Borneol-Dehydrogenase einen Aminosäure-Austausch der Aminosäure E145 mit einer Aminosäure ausgewählt aus G, H, M, L, S auf, wobei die enantioselektive (+)-Borneol-Dehydrogenase aufweisend einen besonders bevorzugten Aminosäure-Austausch der Aminosäure E145 mit S einen Umsatz von 50 % (bezogen auf racemischen Campher) und ein Enantiomeren-Verhältnis (E(+)) von mindestens 7 (E(+)) aufweist.

In einer besonders bevorzugten Ausführungsform weist die erfindungsgemäße enantioselektive (+)-Borneol-Dehydrogenase einen Aminosäure-Austausch der Aminosäure Y188 mit einer Aminosäure ausgewählt aus L, I, V, T, A auf, wobei die enantioselektive (+)-Borneol-Dehydrogenase aufweisend einen besonders bevorzugten Aminosäure-Austausch der Aminosäure Y188 mit A einen Umsatz von mindestens 23 % (bezogen auf racemischen Campher) und ein Enantiomeren-Verhältnis (E(+)) von mehr als 100 (E(+)) aufweist.

In einer besonders bevorzugten Ausführungsform weist die erfindungsgemäße enantioselektive (+)-Borneol-Dehydrogenase einen Aminosäure-Austausch der Aminosäure S146 mit A auf, wobei die erfindungsgemäße enantioselektive (+)-Borneol-Dehydrogenase aufweisend einen Aminosäure-Austausch der Aminosäure S146 mit A einen Umsatz von mindestens 11 % (bezogen auf racemischen Campher) und ein Enantiomeren-Verhältnis (E(+)) von mindestens 6,3 (E(+)) aufweist.

In einer besonders bevorzugten Ausführungsform weist die erfindungsgemäße enantioselektive (+)-Borneol-Dehydrogenase zwei Aminosäure-Austausche auf, wobei der erste Aminosäure-Austausch ein Aminosäure-Austausch der Aminosäure E145 mit einer Aminosäure ausgewählt aus M, L, S ist und der zweite Aminosäure-Austausch ein Aminosäure-Austausch der Aminosäure Y188 mit einer Aminosäure ausgewählt aus T, A, ist, wobei erfindungsgemäß besonders bevorzugt die erfindungsgemäße enantioselektive (+)-Borneol-Dehydrogenase aufweisend zwei Aminosäure-Austasche Aminosäure-Austausche der Aminosäure E145 mit L und der Aminosäure Y188 mit T einen besonders bevorzugten Umsatz von mindestens 16 % (bezogen auf racemischen Campher) und ein besonders bevorzugtes Enantiomeren-Verhältnis (E(+)) von mehr als 100 (E(+)) aufweist.

In einer besonders bevorzugten Ausführungsform weist die erfindungsgemäße enantioselektive (+)-Borneol-Dehydrogenase zwei Aminosäure-Austausche auf, wobei der erste Aminosäure-Austausch ein Aminosäure-Austausch der Aminosäure S146 mit A ist und der zweite Aminosäure-Austausch ein Aminosäure-Austausch der Aminosäure Y188 mit einer Aminosäure ausgewählt aus T, A ist, wobei die erfindungsgemäße enantioselektive (+)-Borneol-Dehydrogenase aufweisend zwei Aminosäure-Austasche besonders bevorzugt einen Aminosäure-Austausch der Aminosäure S146 mit A und einen zweiter Aminosäure-Austausch der Aminosäure Y188 mit T einen besonders bevorzugten Umsatz von mindestens 40 % (bezogen auf racemischen Campher) und ein besonders bevorzugtes Enantiomeren-Verhältnis (E(+)) von mehr als 100 (E(+)) aufweist.

In bevorzugter Ausführungsform betrifft die vorliegende Erfindung eine enantioselektive (+)-Borneol-Dehydrogenase, die einen $K_M$-Wert von mindestens 0,14, mindestens 0,28, mindestens 0,5, mindestens 0,8, mindestens 1,0, mindestens 1,28 (jeweils bezogen auf (-)-Borneol in mM) und einen $K_M$-Wert von höchstens 0,09, höchstens 0,2, höchstens 0,33, höchstens 0,4, höchstens 0,45 (jeweils bezogen auf (+)-Borneol jeweils in mM) aufweist.

In bevorzugter Ausführungsform betrifft die vorliegende Erfindung eine enantioselektive (+)-Borneol-Dehydrogenase, die einen Kcat-Wert von höchstens 0,70, höchstens 0,5, höchstens 0,3, höchstens 0,2, höchstens 0,1, höchstens 0,061 (bezogen auf (-)-Borneol in $sec^{-1}$) und einen $K_{Cat}$-Wert von mindestens 0,49, mindestens 0,7, mindestens 1,0, mindestens 1,5, mindestens 1,97, mindestens 2,5, mindestens 3,0, mindestens 3,59 (bezogen auf (+)-Borneol in $sec^{-1}$) aufweist.

Erfindungsgemäß betrifft die vorliegende Erfindung auch ein Polynukleotid (also ein Polynukleotidmolekül), insbesondere Polynukleotid-Sequenz, insbesondere DNA-Sequenz, kodierend eine erfindungsgemäße enantioselektive Borneol-Dehydrogenase, insbesondere (+)-Borneol-Dehydrogenase.

Das erfindungsgemäße Polynukleotid kann zusätzlich, vorzugsweise im Leseraster zu der die erfindungsgemäße Borneol-Dehydrogenase-codierende DNA-Sequenz, weitere DNA-Sequenzen aufweisen, die beispielsweise Signalpeptide, insbesondere Sezernierungssignale, kodieren. Derartige zusätzliche DNA-Sequenzen können auch Aminosäuren oder Aminosäuresequenzen kodieren, die eine Aufreinigung erfindungsgemäßer Borneol-Dehydrogenasen, insbesondere (+)-Borneol-Dehydrogenase, ermöglichen oder erleichtern.

Erfindungsgemäß betrifft die vorliegende Erfindung auch Vektoren, insbesondere Plasmide, enthaltend ein erfindungsgemäßes Polynukleotid, insbesondere Polynukleotid-Sequenz.

In besonders bevorzugter Ausführungsform kann der Vektor zusätzlich zu dem erfindungsgemäßen Polynukleotid weitere strukturelle und/oder funktionelle Einheiten aufweisen, beispielsweise regulatorische Elemente wie Promotoren, Enhancer, Terminatoren oder andere funktionelle Einheiten wie Resistenzgene oder Linker.

Erfindungsgemäß betrifft die vorliegende Erfindung auch eine Wirtszelle, enthaltend einen erfindungsgemäßen Vektor, insbesondere ein Plasmid.

In besonders bevorzugter Ausführungsform kann die Wirtszelle eine eukaryotische oder prokaryotische Wirtszelle sein. In einer besonders bevorzugten Ausführungsform kann die prokaryotische Wirtszelle eine bakterielle Wirtszelle sein, zum Beispiel E. coli. In besonders bevorzugter Ausführungsform kann die eukaryotische Wirtszelle eine Tierzelle, Pflanzenzelle oder Pilzzelle sein.

Erfindungsgemäß betrifft die vorliegende Erfindung auch ein Verfahren zur Herstellung einer erfindungsgemäßen enantioselektiven Borneol-Dehydrogenase, insbesondere (+)-Borneol-Dehydrogenase, umfassend die folgenden Verfahrensschritte:

l) Bereitstellen einer erfindungsgemäßen Wirtszelle,
m) Kultivieren der in Verfahrensschritt 1) bereitgestellten Wirtszelle in einem Kulturmedium unter Bedingungen, die die Expression einer erfindungsgemäßen enantioselektiven Borneol-Dehydrogenase, insbesondere (+)-Borneol-Dehydrogenase, ermöglichen und
n) Erhalten der in Verfahrensschritt m) exprimierten enantioselektiven Borneol-Dehydrogenase, insbesondere (+)-Borneol-Dehydrogenase.

In einer besonders bevorzugten Ausführungsform der vorliegenden Erfindung sind die Wirtszelle und das Kulturmedium sowie die Bedingungen, die eine Expression einer erfindungsgemäßen enantioselektiven Borneol-Dehydrogenase, insbesondere (+)-Borneol-Dehydrogenase, ermöglichen so auszuführen, dass die enantioselektive Borneol-Dehydrogenase, insbesondere (+)-Borneol-Dehydrogenase, von der Wirtszelle sezerniert und im Kulturmedium erhalten wird, woraus diese durch an sich übliche Methoden isoliert werden kann.

In einer weiteren bevorzugten Ausführungsform kann vorgesehen sein, die Wirtszelle und das Kulturmedium sowie die Bedingungen zur Expression der erfindungsgemäßen enantioselektiven Borneol-Dehydrogenase, insbesondere (+)-Borneol-Dehydrogenase, so auszuführen, dass die Borneol-Dehydrogenase, insbesondere (+)-Borneol-Dehydrogenase, intrazellulär verbleibt und durch anschließende die Wirtszelle aufschließende Verfahrensschritte, beispielsweise Druck- oder Scherkräfte verwendende Verfahren, Lyse durch Detergenzien, osmotischer Schock, Ultraschall, elektrisches Aufschlussverfahren oder andere an sich übliche Verfahren, freigesetzt und sodann in Schritt n) gewonnen wird, insbesondere isoliert werden kann.

Im Zusammenhang mit der vorliegenden Erfindung wird unter dem Begriff "Erhalten einer Substanz oder Zusammensetzung" verstanden, dass die in einem vorhergehenden Verfahrensschritt durch Umsetzung von Reaktanden gewonnene Substanz oder Zusammensetzung aus dem jeweiligen Kultur- oder Reaktionsmedium oder Lösungsmittel heraus verfügbar gemacht wird, insbesondere aus diesem isoliert wird, insbesondere ist ein Erhalten einer Zusammensetzung daher als ein Aufkonzentrieren, insbesondere Isolieren, der gewünschten Zusammensetzung oder Substanz zu verstehen. Die dazu eingesetzten Verfahren können physikalische, chemische Verfahren oder biologische Verfahren sein.

Im Zusammenhang mit der vorliegenden Erfindung wird unter einer Aminosäure mit einer sauren Funktionalität eine Aminosäure ausgewählt aus Asparaginsäure (D) und Glutaminsäure (E) verstanden.

Im Zusammenhang mit der vorliegenden Erfindung wird unter einer Aminosäure mit einer basischen Funktionalität eine Aminosäure ausgewählt aus Lysin (K), Arginin (R), Histidin (H) und Pyrolysin (O) verstanden.

Im Zusammenhang mit der vorliegenden Erfindung wird unter einer Aminosäure mit einer polaren Funktionalität eine Aminosäure ausgewählt aus Serin (S), Cystein (C), Selenocystein (U), Threonin (T), Asparagin (N) und Glutamin (Q) verstanden.

Im Zusammenhang mit der vorliegenden Erfindung wird unter einer Aminosäure mit einer unpolaren Funktionalität eine unpolare Aminosäure ausgewählt aus Glycin (G), Alanin (A), Valin (V), Leucin (L), Isoleucin (I), Phenylalanin (F), Tryptophan (W), Prolin (P) und Methionin (M) verstanden.

Im Zusammenhang mit der vorliegenden Erfindung wird unter einer Aminosäure mit einer phenolischen Funktionalität die Aminosäure Tyrosin (Y) verstanden.

Im Zusammenhang mit der vorliegenden Erfindung wird unter "Enantiomeren-Anreicherung" oder "Enantiomeren-Überschuss" einer Enantiomeren-Zusammensetzung aus einem gewünschten Enantiomer 1 und einem ungewünschten Enantiomer 2 der relative Anteil des gewünschten Enantiomers 1 der Enantiomeren-Zusammensetzung verstanden und über nachfolgende Gleichung berechnet:

$$ee = \frac{|m_1 - m_2|}{m_1 + m_2},$$

wobei *ee* den relativen Enantiomeren-Anteil, $m_1$ den Massenanteil des gewünschten Enantiomers 1 und $m_2$ den Massenanteil des ungewünschten Enantiomers 2 darstellt. Erfindungsgemäß bevorzugt ist eine Zusammensetzung mit $ee \geq 0.99$ als besonders bevorzugt angesehen und liegt als nahezu Enantiomeren-reine Zusammensetzung des gewünschten Enantiomers vor.

Im Zusammenhang mit der vorliegenden Erfindung wird unter "gewünschtes Enantiomer" das Enantiomer verstanden, welches nicht bevorzugt von der Borneol-Dehydrogenase, insbesondere (+)-Borneol-Dehydrogenase, umgesetzt wird und unter "ungewünschtes Enantiomer" das Enantiomer, welches bevorzugt von der Borneol-Dehydrogenase, insbesondere (+)-Borneol-Dehydrogenase, umgesetzt wird verstanden.

In einer bevorzugten Ausführungsform ist das gewünschte Enantiomer das 1R-(+)-Campher-Enantiomer. In einer weiteren bevorzugten Ausführungsform ist das gewünschte Enantiomer das 1S-(-)-Campher-Enantiomer.

In einer besonders bevorzugten Ausführungsform ist die erfindungsgemäß bereitgestellte enantioselektive Borneol-Dehydrogenase eine (+)-Borneol-Dehydrogenase, sodass das gewünschte Enantiomer ein 1S-(-)-Campher-Enantiomer ist.

In einer besonders bevorzugten Ausführungsform ist die erfindungsgemäße bereitgestellte enantioselektive Borneol-Dehydrogenase eine (-)-Borneol-Dehydrogenase, sodass das gewünschte Enantiomer ein 1R-(+)-Campher-Enantiomer ist.

Im Zusammenhang mit vorliegender Erfindung wird unter "$ee_s$" der relative Anteil des ungewünschten Enantiomers in der umgesetzten Zusammensetzung, bezogen auf das gewünschte Enantiomer und das ungewünschte Enantiomer in der umgesetzten Zusammensetzung, verstanden. Erfindungsgemäß bevorzugt ist $ee_s \geq 0.99$, da somit nahezu allein das Gesamte ungewünschte Enantiomer umgesetzt wurde und das gewünschte Enantiomer nahezu vollständig, insbesondere vollständig, in der umgesetzten Zusammensetzung vorliegt.

Im Zusammenhang mit der vorliegenden Erfindung wird unter "$ee_p$" der relative Anteil des gewünschten Enantiomers in der umgesetzten Zusammensetzung, bezogen auf das gewünschte Enantiomer und das ungewünschten Enantiomer in der umgesetzten Zusammensetzung, verstanden. Erfindungsgemäß bevorzugt ist $ee_P \geq 0.99$, da somit nahezu allein das Gesamte ungewünschte Enantiomer umgesetzt wurde und das gewünschte Enantiomer nahezu vollständig, insbesondere vollständig in der umgesetzten Zusammensetzung vorliegt.

Im Zusammenhang mit der vorliegenden Erfindung wird der "Umsatz" über folgende Gleichung berechnet:

$$Umsatz = ee_s/(ee_s + ee_p).$$

Im Zusammenhang mit der vorliegenden Erfindung wird unter "Enantiomeren-Verhältnis" das Enantiomeren-Verhältnis verstanden, welches in Verbindung mit enzymatischer Racematspaltung gebildet wird.

Im Zusammenhang mit der vorliegenden Erfindung wird das "Enantiomeren-Verhältnis" über folgende Gleichung berechnet:

$$E = \ln((ee_p * (1 - ee_s))/(ee_p + ee_s))/\ln((ee_p * (1 + ee_s))/(ee_p + ee_s)),$$

wobei *E* das Enantiomeren-Verhältnis, $ee_s$ den relativen Anteil des ungewünschten Enantiomers in der umgesetzten Zusammensetzung, bezogen auf das gewünschte Enantiomer und das ungewünschte Enantiomer in der umgesetzten Zusammensetzung und $ee_p$ den relativen Anteil des gewünschten Enantiomers in der umgesetzten Zusammensetzung, bezogen auf das gewünschte Enantiomer und das ungewünschte Enantiomer in der umgesetzten Zusammensetzung darstellt.

Erfindungsgemäß bevorzugt ist $E \geq 100$, da somit das gewünschte Enantiomer allein oder nahezu allein in der umgesetzten Zusammensetzung vorliegt.

Im Zusammenhang mit der vorliegenden Erfindung bezieht sich eine Angabe zum Enantiomeren-Verhältnis (E) und Umsatz auf die zur Umsetzung von Campher befähigte erfindungsgemäße enantioselektive Borneol-Dehydrogenase, insbesondere E(+) und entsprechender Umsatz auf eine erfindungsgemäße enantioselektive (+)-Borneol-Dehydrogenase, die insbesondere bevorzugt befähigt ist, 1R-(+)-Campher zu (+)-Borneol umzusetzen und insbesondere E(-) und entsprechender Umsatz auf eine erfindungsgemäße enantioselektive (-)-Borneol-Dehydrogenase, die insbesondere bevorzugt befähigt ist 1S-(-)-Campher zu (-)-Borneol umzusetzen.

In besonders bevorzugter Ausführungsform werden das Enantiomeren-Verhältnis E(+) beziehungsweise E(-) sowie die entsprechenden Umsätze der jeweiligen (+)- beziehungsweise (-)-Borneol-Dehydrogenase in einem eine Campher-Reduktion ermöglichenden Reaktionsgemisch bestimmt, insbesondere gemäß der in Beispiel 2, insbesondere Beispiel 2 e), angegebenen Verfahrensweise bestimmt.

Im Zusammenhang mit der vorliegenden Erfindung wird unter "Campher" ein racemisches Campher-Gemisch und ein einen Enantiomeren-Überschuss von 1R-(+)-Campher oder 1S-(-)-Campher aufweisendes Campher-Gemisch verstanden.

Im Zusammenhang mit der vorliegenden Erfindung wird unter "racemischer Campher" und/oder "racemisches Campher-Gemisch" und/oder "Campher-Racemat" eine Zusammensetzung aus verschiedenen chemischen enantiomeren Campher-Verbindungen, insbesondere zwei Campher-Verbindungen verstanden, die in einem äquimolaren Verhältnis zueinander vorliegen, insbesondere in einem 1:1-Stoffmengenverhältnis.

Im Zusammenhang mit der vorliegenden Erfindung wird unter "Verbindung" ein Molekül oder mehrere identische Moleküle verstanden.

Im Zusammenhang mit der vorliegenden Erfindung wird unter "chiral" und/oder "Chiralität" eine Verbindung verstanden, die nicht zur Deckungsgleichheit mit ihrem Spiegelbild gebracht werden kann.

Im Zusammenhang mit der vorliegenden Erfindung wird unter "Enantiomer" eine von mindestens zwei chiralen Verbindungen gleicher Konstitution verstanden, wobei die mindestens zwei chiralen Verbindungen sich wie Bild und Spiegelbild verhalten.

Im Zusammenhang mit der vorliegenden Erfindung wird unter "Enantiomeren-Überschuss von 1R-(+)-Campher oder 1S-(-)-Campher aufweisendes Gemisch" ein Gemisch verstanden, das ein nicht äquimolares Stoffmengen-Verhältnis der Campher-Enantiomere aufweist.

Ein Enantiomeren-Überschuss von 1R-(+)-Campher oder 1S(-)-Campher aufweisendes Gemisch ist im Zusammenhang mit der vorliegenden Erfindung ein Ausgangsmaterial für die erfindungsgemäße Racematspaltung in Verfahrensschritt b).

Im Zusammenhang mit der vorliegenden Erfindung ist ein einen Enantiomeren-Überschuss von 1R-(+)-Campher-aufweisendes Gemisch ein Gemisch, enthaltend 99 bis 51 mol-% 1R-(+)-Campher zu 1 bis 49 mol-% 1S-(-)-Campher (bezogen auf beide Campher-Enantiomere).

Im Zusammenhang mit der vorliegenden Erfindung ist ein einen Enantiomeren-Überschuss von 1S-(-)-Campher-aufweisendes Gemisch ein Gemisch, enthaltend 99 bis 51 mol-% 1S-(-)-Campher zu 1 bis 49 mol-% 1R-(+)-Campher (bezogen auf beide Campher-Enantiomere).

Im Zusammenhang mit der vorliegenden Erfindung ist in bevorzugter Ausführungsform ein einen Enantiomeren-Überschuss von 1S-(-)-Campher oder 1R-(+)-Campher-aufweisendes Gemisch ein Gemisch, in dem höchstens 10 mol-%, höchstens 5 mol-%, höchstens 1 mol-%, höchstens 0,5 mol-%, höchstens 0,1 mol-% (bezogen auf das Gemisch) Borneol, insbesondere kein Borneol-Enantiomer vorhanden ist.

Im Zusammenhang mit der vorliegenden Erfindung ist eine 1R-(+)-Campher-Enantiomeren-angereicherte Zusammensetzung oder eine 1S-(-)-Campher-Enantiomeren-angereicherte Zusammensetzung das Produkt einer erfindungsgemäßen Racematspaltung in Verfahrensschritt b).

Im Zusammenhang mit der vorliegenden Erfindung ist eine 1S-(-)-Campher-Enantiomeren-angereicherte Zusammensetzung das Produkt einer erfindungsgemäßen Racematspaltung in Verfahrensschritt b), insbesondere durch eine enantioselektive Borneol-Dehydrogenase, insbesondere (+)-Borneol-Dehydrogenase.

Im Zusammenhang mit der vorliegenden Erfindung wird in besonders bevorzugter Ausführungsform unter einer "1R-(+)-Campher-Enantiomeren-angereicherten Zusammensetzung" eine Zusammensetzung verstanden, die nach Umsetzung mit einer enantioselektiven Borneol-Dehydrogenase einen höheren Anteil an 1R-(+)-Campher-Enantiomer im Vergleich zum 1S-(-)-Campher-Enantiomer aufweist als im für die Umsetzung mit der enantioselektiven Borneol-Dehydrogenase eingesetzten Ausgangsmaterial, nämlich Campher.

Im Zusammenhang mit der vorliegenden Erfindung wird unter einer "1S-(-)-Campher-Enantiomeren-angereicherten Zusammensetzung" eine Zusammensetzung verstanden, die nach Umsetzung mit einer enantioselektiven Borneol-Dehydrogenase, insbesondere (+)-Borneol-Dehydrogenase, einen höheren Anteil an 1S-(-)-Campher-Enantiomer im Vergleich zum 1R-(+)-Campher-Enantiomer aufweist als im für die Umsetzung mit der enantioselektiven Borneol-Dehydrogenase eingesetzten Ausgangsmaterial, nämlich Campher.

Im Zusammenhang mit der vorliegenden Erfindung wird in besonders bevorzugter Ausführungsform unter "1R-(+)-Campher oder 1S-(-)-Campher-Enantiomeren-angereicherter Zusammensetzung" eine Zusammensetzung enthaltend 1R-(+)-Campher oder 1S-(-)-Campher, insbesondere 1S-(-)-Campher, verstanden, in der die beiden Enantiomere nicht äquimolar vorliegen, das heißt nicht in einem 1:1-Stoffmengenverhältnis, insbesondere die nur 1S-(-)-Campher oder 1R-(+)-Campher aufweist.

Im Zusammenhang mit der vorliegenden Erfindung wird unter einer "Campher-Enantiomeren-angereicherten Zusammensetzung" eine Zusammensetzung verstanden, die 1 bis 99 mol-% Campher-Enantiomere und 99 bis 1 mol-% Borneol-Enantiomere (bezogen auf alle Campher- und Borneol-Enantiomere in der Enantiomeren-angereicherten Zusammensetzung) enthalten.

Im Zusammenhang mit der vorliegenden Erfindung wird bevorzugt unter "1R-(+)-Campher- oder (1S)-(-)-Campher-Enantiomeren-angereicherten Zusammensetzung" auch eine 1R-(+)- oder 1S-(-)-Campher angereicherte Zusammensetzung verstanden, die die beiden genannten enantiomeren Verbindungen umfasst, insbesondere hauptsächlich umfasst, insbesondere mindestens 50 %, insbesondere mindestens 60 %, insbesondere mindestens 70 %, insbesondere mindestens 80 %, insbesondere mindestens 90 %, insbesondere mindestens 95 %, insbesondere mindestens 99 % umfasst (bezogen auf Massentrockensubstanz der enantiomeren Verbindungen zu Massentrockensubstanz der Zusammensetzung), insbesondere aus den enantiomeren Verbindungen besteht.

Im Zusammenhang mit der vorliegenden Erfindung wird unter "enantioselektiv" das Reaktionsgeschwindigkeits-Verhältnis zweier Enantiomere bei deren Umsetzung verstanden. Eine hohe Enantioselektivität beschreibt daher eine Umsetzung, bei der ein Enantiomer bevorzugt gegenüber den anderen Enantiomeren umgesetzt wird, insbesondere schneller.

Im Zusammenhang mit der vorliegenden Erfindung wird unter "enantioselektiver Borneol-Dehydrogenase" eine erfindungsgemäße Borneol-Dehydrogenase, insbesondere (+)-Borneol-Dehydrogenase, verstanden, die enantioselektiv Campher, insbesondere 1R-(+)-Campher, umsetzt, sowie, in optionaler Ausführungsform, alle Enzyme, die in einem eine Campher-Reduktion ermöglichenden Reaktionsgemisch, insbesondere unter den Bedingungen gemäß Beispiel 2, insbesondere Beispiel 2 e), zu einem vergleichbaren Enantiomeren-Verhältnis und/oder Umsatz führen, vorzugsweise einem Enantiomeren-Verhältnis von 6,0 (E(+)), insbesondere 7,0 (E(+)), insbesondere 12,1 (E(+)), insbesondere 30,5 (E(+)), insbesondere 72,6 (E(+)), insbesondere mehr als 100 (E(+)) oder einem Umsatz und einem Enantiomeren-Verhältnis von mehr als 2 % und 30,5 (E(+)), insbesondere mehr als 5 % und 6 (E(+)), insbesondere mehr als 17 % und 12,1 (E(+)), insbesondere mehr als 10 % und 72,6 (E(+)), insbesondere mehr als 11 % und mehr als 100 (E(+)) (bezogen auf racemischen Campher), insbesondere bezogen auf das Enantiomeren-Verhältnis und/oder Umsatz einer unter identischen Reaktionsbedingungen eingesetzten erfindungsgemäßen Borneol-Dehydrogenase, insbesondere (+)-Borneol-Dehydrogenase, mit einer der Sequenznummern SEQ ID No. 1, 3, 5, 7, 9, 11, 13, 15, 17, 19, 21, 23, 25, 27, 29, 31, 33, 35, 37 oder 39.

Im Zusammenhang mit der vorliegenden Erfindung wird unter einer "enantioselektiven (+)-Borneol-Dehydrogenase" eine enantioselektive Borneol-Dehydrogenase verstanden, die enantioselektiv, bevorzugt allein, 1R-(+)-Campher-Enantiomer zu (+)-Borneol umsetzt.

Im Zusammenhang mit der vorliegenden Erfindung wird unter einer "enantioselektiven (-)-Borneol-Dehydrogenase" eine enantioselektive Borneol-Dehydrogenase verstanden, die enantioselektiv, vorzugsweise das 1S-(-)-Campher-Enantiomer zu (-)-Borneol umsetzt.

Im Zusammenhang mit der vorliegenden Erfindung wird unter der Abkürzung "NAD" Nicotinsäureamid-Adenin-Dinukleotid verstanden. Im Zusammenhang mit der vorliegenden Erfindung wird unter "NADH" die reduzierte Form von NAD verstanden. Im Zusammenhang mit der vorliegenden Erfindung wird unter der Abkürzung "NADP" Nicotinsäureamid-Adenin-Dinukleotid-Phosphat verstanden. Im Zusammenhang mit der vorliegenden Erfindung wird unter "NADPH" die reduzierte Form von NADP verstanden. Im Zusammenhang mit der vorliegenden Erfindung wird unter "NADH/NADPH-Analogon" eine chemische Verbindung zum Beispiel Thionicotinsäureamid-Adenin-Dinukleotid (S-NAD), Nicotinsäure-Adenin-Dinukleotid (O-NAD), Nicotinsäureamid-Hypoxanthin-Dinukleotid (NHD), Nicotinsäureamid-Guanin-Dinukleotid, oder weitere Verbindungen, die eine ähnliche, bevorzugt gleiche Aktivität wie NADH und/oder NADPH aufweisen, verstanden.

Im Zusammenhang mit der vorliegenden Erfindung wird der in Verfahrensschritt a) optional bereitgestellte "reduzierende Co-Faktor" auch als erstes Substrat bezeichnet.

Im Zusammenhang mit der vorliegenden Erfindung wird unter "Regenerationssystem" ein System verstanden, das zur Rückgewinnung des reduzierenden Co-Faktors dient, insbesondere zur Reduktion des oxidierten Co-Faktors.

Im Zusammenhang mit der vorliegenden Erfindung wird unter einem "Regenerationssystem" ein System verstanden, aufweisend ein zur Reduktion befähigtes Enzym, insbesondere ein zur Reduktion eines zweiten Substrats befähigtes Enzym, insbesondere eine Dehydrogenase, insbesondere eine Glycosyl- oder Formiat-Dehydrogenase, insbesondere eine Glucose- oder Formiat-Dehydrogenase und ein zweites Substrat, insbesondere ein von dem befähigten Enzym zu reduzierendes zweites Substrat, insbesondere einen Zucker, insbesondere einen Einfachzucker wie Glucose, oder eine Säure, insbesondere Ameisensäure.

Im Zusammenhang mit der vorliegenden Erfindung sind sämtliche Positionsangaben von Aminosäuren im Einlettercode bezogen auf die Aminosäuresequenz der Wildtyp-Borneol-Dehydrogenase aus Pseudomonas putida (ATCC17453), so wie sie in SEQ ID No. 1 angegeben ist.

Im Zusammenhang mit der vorliegenden Erfindung werden spezifische Mutanten der erfindungsgemäßen enantioselektiven Borneol-Dehydrogenase, insbesondere (+)-Borneol-Dehydrogenase, durch Angabe der in der SEQ ID No. 1 ausgetauschten Aminosäure im Einlettercode, darauffolgend die Positionsangabe, in Bezug auf SEQ ID No. 1, an der sich die ausgetauschte Aminosäure befindet und anschließend die Angabe der die ausgetauschte Aminosäure ersetzenden Aminosäure mit anderer Funktionalität im Einlettercode gekennzeichnet. So bedeutet beispielsweise die Bezeichnung S146A, dass in der SEQ ID No. 1 mit der Wildtyp-Borneol-Dehydrogenase-Sequenz die an der Position 146 vorhandene Aminosäure S ausgetauscht wird durch eine Aminosäure anderer Funktionalität, nämlich A.

In einer bevorzugten Ausführungsform setzt die in Verfahrensschritt a) bereitgestellte enantioselektive Borneol-Dehydrogenase enantioselektiv 1R-(+)-Campher um.

In einer bevorzugten Ausführungsform wird das in dem in Verfahrensschritt c) erhaltene Gemisch aus 1S-(-)-Campher und (+)-Borneol in einem Verfahrensschritt d) mittels selektiver chemischer Umsetzung oder einer physikalischen Auftrennmethode in mindestens eine Campher- und eine Borneol-haltige Fraktion aufgetrennt, insbesondere mit mindestens einer Chromatographie- oder Destillationsmethode oder Kristallisation.

In einer bevorzugten Ausführungsform wird das in Verfahrensschritt d) erhaltene (+)-Borneol in einem Verfahrensschritt e) zu 1R-(+)-Campher umgesetzt.

In einer bevorzugten Ausführungsform wird die aus den Verfahrensschritten a) bis c) erhaltene (1S)-(-)-Campher-Enantiomeren-angereicherte Zusammensetzung in einem Verfahrensschritt f) zu 1S-(+)-Campher-10-Sulfonsäure umgesetzt, wobei der Verfahrensschritt f) folgende Schritte f1) bis f4) umfasst:

f1) Zugeben von Schwefelsäure zu der (1S)-(-)-Campher-Enantiomeren-angereicherten Zusammensetzung in einem organischen Lösungsmittel, insbesondere Acetanhydrid,

f2) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und

f3) Erhalten einer Zusammensetzung von 1S-(+)-Campher-10-Sulfonsäure und (+)-Borneol, wobei vorzugsweise anschließend erfolgt ein

f4) Auftrennen der erhaltenen Zusammensetzung von 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

In einer bevorzugten Ausführungsform wird das in Verfahrensschritt f4) erhaltene (+)-Borneol in einem Verfahrensschritt g) zu 1R-(+)-Campher umgesetzt.

Erfindungsgemäß betrifft die vorliegende Erfindung auch ein Verfahren zur Herstellung von 1S-(+)-Campher-10-Sulfonsäure umfassend die folgenden Verfahrensschritte i1) bis i5):

i1) Durchführen der Verfahrensschritte gemäß Anspruch 1 Verfahrensschritte a) bis c),

i2) Zugeben von Schwefelsäure zu der (1S)-(-)-Campher-Enantiomeren-angereicherten Zusammensetzung in einem organischen Lösungsmittel, insbesondere Acetanhydrid,

i3) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und

i4) Erhalten einer Zusammensetzung von 1S-(+)-Campher-10-Sulfonsäure und (+)-Borneol, wobei vorzugsweise anschließend erfolgt ein

i5) Auftrennen der erhaltenen Zusammensetzung von 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

In einer bevorzugten Ausführungsform wird das aus Verfahrensschritt i5) erhaltene (+)-Borneol in einem Verfahrensschritt j) zu 1R-(+)-Campher umgesetzt.

Weitere bevorzugte Ausführungsformen der vorliegenden Erfindung ergeben sich aus den Unteransprüchen.

Die vorliegende Erfindung wird in den folgenden Beispielen näher erläutert, wobei diese nicht einschränkend zu verstehen sind.

Das Sequenzprotokoll zeigt:
SEQ ID No. 1 stellt die Aminosäuresequenz der Wildtyp Borneol-Dehydrogenase aus Pseudomonas putida (ATCC17453) dar und SEQ ID No. 2 die dazugehörige DNA-Sequenz. Pseudomonas putida (ATCC17453) weist eine Borneol-Dehydrogenase, insbesondere (+)-Borneol-Dehydrogenase, auf, wobei das erfindungsgemäß klonierte Borneol-Dehydrogenase-Gen eine Polypeptidkette von 260 Aminosäuren kodiert, welche bevorzugt eine Masse von 27,5 kDa und einen pI von 5,35 aufweist.

SEQ ID No. 3 stellt die Aminosäuresequenz einer enantioselektiven Borneol-Dehydrogenase, insbesondere (+)-Borneol-Dehydrogenase, mit einem Aminosäure-Austausch der Aminosäure S146 mit A dar (S146A) und SEQ ID No. 4 die dazugehörige DNA-Sequenz. SEQ ID No. 5 stellt die Aminosäuresequenz einer enantioselektiven Borneol-Dehydrogenase, insbesondere (+)-Borneol-Dehydrogenase, mit einem Aminosäure-Austausch der Aminosäure E145 mit G (E145G) dar und SEQ ID No. 6 die dazugehörige DNA-Sequenz. SEQ ID No. 7 stellt die Aminosäuresequenz einer enantioselektiven Borneol-Dehydrogenase, insbesondere (+)-Borneol-Dehydrogenase, mit einem Aminosäure-Austausch der Aminosäure E145 mit H (E145H) dar und SEQ ID No. 8 die dazugehörige DNA-Sequenz. SEQ ID No. 9 stellt die Aminosäuresequenz einer enantioselektiven Borneol-Dehydrogenase, insbesondere (+)-Borneol-Dehydrogenase, mit einem Aminosäure-Austausch der Aminosäure E145 mit M (E145M) dar und SEQ ID No. 10 die dazugehörige DNA-Sequenz. SEQ ID No. 11 stellt die Aminosäuresequenz einer enantioselektiven Borneol-Dehydrogenase, insbesondere (+)-Bomeol-Dehydrogenase, mit einem Aminosäure-Austausch der Aminosäure E145 mit L (E145L) dar und SEQ ID No. 12 die dazugehörige DNA-Sequenz. SEQ ID No. 13 stellt die Aminosäuresequenz einer enantioselektiven Borneol-Dehydrogenase, insbesondere (+)-Borneol-Dehydrogenase, mit einem Aminosäure-Austausch der Aminosäure E145 mit S (E145S) dar und SEQ ID No. 14 die dazugehörige DNA-Sequenz. SEQ ID No. 15 stellt die Aminosäuresequenz einer enantioselektiven Borneol-Dehydrogenase, insbesondere (+)-Borneol-Dehydrogenase, mit einem Aminosäure-Austausch der Aminosäure Y188 mit L (Y188L) dar und SEQ ID No. 16 die dazugehörige DNA-Sequenz. SEQ ID No. 17 stellt die Aminosäuresequenz einer enantioselektiven Borneol-Dehydrogenase, insbesondere (+)-Borneol-Dehydrogenase, mit einem Aminosäure-Austausch der Aminosäure Y188 mit I (Y188I) dar und SEQ ID No. 18 die dazugehörige DNA-Sequenz. SEQ ID No. 19 stellt die Aminosäuresequenz einer enantioselektiven Borneol-Dehydrogenase, insbesondere (+)-Borneol-Dehydrogenase, mit einem Aminosäure-Austausch der Aminosäure Y188 mit V (Y188V) dar und SEQ ID No. 20 die dazugehörige DNA-Sequenz. SEQ ID No. 21 stellt die Aminosäuresequenz einer enantioselektiven Borneol-Dehydrogenase, insbesondere (+)-Borneol-Dehydrogenase, mit einem Aminosäure-Austausch der Aminosäure Y188 mit T (Y188T) dar und SEQ ID No. 22 die dazugehörige DNA-Sequenz. SEQ ID No. 23 stellt die Aminosäuresequenz einer enantioselektiven Borneol-Dehydrogenase, insbesondere (+)-Borneol-Dehydrogenase, mit einem Aminosäure-Austausch der Aminosäure Y188 mit A (Y188A) dar und SEQ ID No. 24 die dazugehörige DNA-Sequenz. SEQ ID No. 25 stellt die Aminosäuresequenz einer enantioselektiven Borneol-Dehydrogenase, insbesondere (+)-Borneol-Dehydrogenase, mit zwei Aminosäure-Austausche der Aminosäure E145 mit M (E145M) und der Aminosäure Y188 mit A (Y188A) dar und SEQ ID No. 26 die dazugehörige DNA-Sequenz. SEQ ID No. 27 stellt die Aminosäuresequenz einer enantioselektiven Borneol-Dehydrogenase, insbesondere (+)-Borneol-Dehydrogenase, mit zwei Aminosäure-Austausche der Aminosäure E145 mit M (E145M) und der Aminosäure Y188 mit T (Y188T) dar und SEQ ID No. 28 die dazugehörige DNA-Sequenz. SEQ ID No. 29 stellt die Aminosäuresequenz einer enantioselektiven Borneol-Dehydrogenase, insbesondere (+)-Borneol-Dehydrogenase, mit zwei Aminosäure-Austausche der Aminosäure E145 mit L (E145L) und der Aminosäure Y188 mit T (Y188T) dar und SEQ ID No. 30 die dazugehörige DNA-Sequenz. SEQ ID No. 31 stellt die Aminosäuresequenz einer enantioselektiven Borneol-Dehydrogenase, insbesondere (+)-Borneol-Dehydrogenase, mit zwei Aminosäure-Austausche der Aminosäure E145 mit L (E145L) und der Aminosäure Y188 mit A (Y188A) dar und SEQ ID No. 32 die dazugehörige DNA-Sequenz. SEQ ID No. 33 stellt die Aminosäuresequenz einer enantioselektiven Borneol-Dehydrogenase, insbesondere (+)-Borneol-Dehydrogenase, mit zwei Aminosäure-Austausche der Aminosäure E145 mit S (E145S) und der Aminosäure Y188 mit T (Y188T) dar und SEQ ID No. 34 die dazugehörige DNA-Sequenz. SEQ ID No. 35 stellt die Aminosäuresequenz einer enantioselektiven Borneol-Dehydrogenase, insbesondere (+)-Borneol-Dehydrogenase, mit zwei Aminosäure-Austausche der Aminosäure E145 mit S (E145S) und der Aminosäure Y188 mit A (Y188A) dar und SEQ ID No. 36 die dazugehörige DNA-Sequenz. SEQ ID No. 37 stellt die Aminosäuresequenz einer enantioselektiven Borneol-Dehydrogenase, insbesondere (+)-Borneol-Dehydrogenase, mit zwei Aminosäure-Austausche der Aminosäure S146 mit A (S146A) und der Aminosäure Y188 mit T (Y188T) dar und SEQ ID No. 38 die dazugehörige DNA-Sequenz. SEQ ID No. 39 stellt die Aminosäuresequenz einer enantioselektiven Borneol-Dehydrogenase, insbesondere (+)-Borneol-Dehydrogenase, mit zwei Aminosäure-Austausche der Aminosäure S146 mit A (S146A) und der Aminosäure Y188 mit A (Y188A) dar und SEQ ID No. 40 die dazugehörige DNA-Sequenz. SEQ ID No. 41 stellt die DNA-Sequenz des Vektors dar. SEQ ID No. 42 stellt die DNA-Sequenz des Primers dar, das für die Reaktion verwendet wurde, dar. SEQ ID No. 43 stellt die DNA-Sequenz der His-markierten Variante des Primers dar.

Die Figuren zeigen:

| | |
|---|---|
| Figur 1 | schematisch die erfindungsgemäße enzymatische Racematspaltung von Campher mittels der erfindungsgemäßen enantioselektiven Borneol-Dehydrogenase, wobei in der oberen Reaktion der racemische Campher mittels einer enantioselektiven (+)-Borneol-Dehydrogenase und in der unteren Reaktion der racemische Campher mittels einer enantioselektive (-)-Borneol-Dehydrogenase gespalten wird, |
| Figur 2 | eine schematische Darstellung der erfindungsgemäßen Verfahrensschritte der Racematspaltung und Sulfonierung und |
| Figur 3 | eine Karte eines erfindungsgemäßen Vektors unter Verwendung folgender Abkürzungen: BDH: Borneol Dehydrogenase MCS: Multiple Cloning Site |
| ori: | Replikationsursprung |
| f1 origin: | Replikationsursprung |
| Kan: | Gen für Kanamycin Resistenz |
| lacI: | Gen für lac Repressor |
| rop: | Gen für rop Protein |

## Beispiele

### Beispiel 1: Herstellung erfindungsgemäßer enantioselektiver (+)-Borneol-Dehydrogenasen

a) Klonierung

Pseudomonas putida (ATCC17453) wurde auf LB-Agar, der 100 ug / ml Ampicillin enthielt, bei 30 °C kultiviert. Ein einzelner Klon wurde ausgewählt und in 10 μl Aquadest (destilliertes Wasser) transferiert und als DNA-Matrize verwendet. Die PCR-Bedingungen für die Amplifikation des BDH-Gens waren 5 Minuten bei 98 °C für die DNA-Denaturierung, gefolgt von 25 Denaturierungszyklen bei 98 °C für 10 Sekunden, Primer-Annealing bei 61 °C für 20 Sekunden und Strangverlängerung bei 72 °C für 30 Sekunden. Ein letzter Inkubationsschritt bei 72 °C für 10 Minuten beendete die Reaktion unter Verwendung von Phusions-DNA-Polymerase.

Das folgende Paar Oligonukleotide wurde für die Reaktion verwendet: 5-GATCCCATGGATGAAACCGCTAGCAGG-3 und 5-GATAGCGGCCGCTCAACCAGACAATCGATTG-3. (SEQ ID No. 42) Für die Erzeugung der His-markierten Variante wurden folgende Primer verwendet: 5-TTGGTCTCGGA TGCACCACCACCACCACCACAAACCGCT AGCAGGT AAAAG-3 und 5-GATAGCGGCCGCTCAACCAGACAATCGATTG-3 (SEQ ID No. 43) . Die PCR-Produkte wurden mit NcoI oder BsaI und NotI verdaut und in mit den gleichen Restriktionsenzymen verdautes pET28a kloniert. Alle Konstrukte wurden anschließend sequenziert.

b) Mutagenese (Verfahrensschritte 1))

Das Wildtyp-Enzym wurde durch ortsgerichtete Mutagenese an den Positionen Q96, H98, E145, S146, N154 und Y188 geändert. Die Primer für die Mutagenese wurden wie beschrieben entworfen (Edelheit et al., BMC Biotechnol.,

9, 2009, 61). Die PCR-Bedingungen waren 2 min bei 95 °C für die DNA-Denaturierung, gefolgt von 16 Denaturierungszyklen bei 95 °C für 45 Sekunden, Primer-Annealing bei 55 °C für 30 Sekunden und Strangverlängerung bei 72 °C für 12 Minuten. Ein letzter Inkubationsschritt bei 72 °C für 10 Minuten beendete die Reaktion unter Verwendung von Pfu-DNA-Polymerase. Danach wurde 1 µl DpnI zu dem Reaktionsgemisch gegeben und 2 Stunden bei 37 °C inkubiert, bevor die Ligation und Transformation in E. coli DH10B durchgeführt wurde. Die erfindungsgemäßen Aminosäure-Austausche wurden, wie aus der nachstehenden Tabelle 1 ersichtlich, durchgeführt.

Tabelle 1 Ausgewählte Aminosäure-Austausche für die ortsgerichtete Mutagenese.

| | |
|---|---|
| Q96: | R, F, Y, W, K, E, N, L, M. |
| H98: | F, Y, W, Q, R, K, E, L, M, N. |
| E145: | M, L, N, I, S, H, K, R, Q, F, Y, W, G |
| S146: | G, A, V, I, L, N, D, E, T. |
| N154: | L, I, M, E, S, H, K, W, Y, F, Q, R. |
| Y188: | W, H, R, Q, F, K, E, L, M, N. |

c) Enzymproduktion (Verfahrensschritt m))

E. coli BL21(DE3) wurden mit dem Vektor pET28a Borneol-Dehydrogenase (Vektor gemäß Figur 3) durch Elektroporation transformiert. Die Transformationslösung wurde auf LB Agar mit 100 µg/ml Kanaymcin ausgestrichen und bei 37 °C über Nacht inkubiert. Eine einzelne Kolonie wurde dann in 50 ml LB Medium mit 100 µg/ml Kanamycin transferiert und unter Schütteln (140 U/min) bei 37 °C über Nacht inkubiert. Aus dieser Vorkultur wurde die Hauptkultur beimpft und auf eine optische Dichte (600 nm) von 0,2 eingestellt. Die Hauptkultur wurde in 200 ml LB mit 100 µg/ml Kanamycin bei 140 U/min und 37 °C so lange inkubiert, bis eine optische Dichte von 0,6 erreicht wurde. Es folgte die Zugabe von 1 mM IPTG. Die Proteinexpression erfolgte anschließend bei 140 U/min und 16 °C über Nacht. Die Kultur wurde bei 3500 × g zentrifugiert und das Zellpellet für die Enzymreinigung verwendet.

d) Enzymreinigung (Verfahrensschritt n))

Alle Reinigungsschritte wurden bei 4 °C durchgeführt. Das Zellpellet wurde in Puffer A resuspendiert, der 50 mM Kaliumphosphatpuffer, pH 8,0, 20 mM Imidazol und 0,5 M Natriumchlorid enthielt. Dann wurden die Zellen in einer French-Presse bei 1350 bar aufgeschlossen. Nach 30-minütiger Zentrifugation bei 30.000 × g wurde die lösliche Fraktion, die BDH enthielt, durch einen 0,2 µm-Sterilfilter geleitet, bevor sie auf eine 1 ml HisTrap FF-Roh-1 ml-Säule geladen wurde. Die Säule wurde zuvor mit Puffer A äquilibriert. Die Säule wurde mit 50 mM Kaliumphosphatpuffer pH 7,5, 50 mM Imidazol und 0,5 M Natriumchlorid, gewaschen, und das BDH-Enzym wurde danach mit 50 mM Kaliumphosphatpuffer pH 7,5, 300 mM Imidazol und 0,5, M Natriumchlorid eluiert. Die BDH-Fraktionen wurden vereinigt und unter Verwendung einer HiPrep 26/10-Entsalzungssäule und 50 mM Kaliumphosphatpuffer pH 7,5, entsalzt. Das Protein konnte mit nur geringen Aktivitätsverlusten mindestens 1 Woche bei 4 °C gelagert werden.

**Beispiel 2: Aktivitätsuntersuchungen der erfindungsgemäßen enantioselektiven (+)-BDH**

a) Analysemethoden

Analyse-Produkte wurden nach der Produktextraktion aus Assays mit äquivalenten Mengen Ethylacetat, das 1 mM Hexylbenzol als internen Standard enthält, analysiert. GC-Analysen wurden mit einem Shimadzu GCMS Model 2010 Q Plus das mit einem MS-Detektor (Single Quad) verbunden war, durchgeführt. Chromatographische Auftrennungen wurden an einer FS-HYDRODEX β-6TBD Kapillarsäule (25 m × 0,25 mm) durchgeführt. 1,0 µl der Probe wurden bei 250 °C eingespritzt (Split-Splitless injector). Die Ofentemperatur wurde bei 60 °C für 8 Minuten gehalten und dann auf 150 °C mit einer Heizrate von 2,0 °C / Minute, und dann auf 200 °C mit einer Heizrate von 45 °C / Minute erhöht und zwei Minuten gehalten. Die Fließgeschwindigkeit innerhalb der Säule betrug 1,9 ml / Minute (Trägergas: Helium). Die Produkte wurden anhand des Vergleichs ihrer Retentionszeiten und Massenspektren mit authentischen Standards bestimmt.

b) Enzymaktivität (enantioselektive Oxidation von Borneol zu Campher)

Das Oxidationsreaktionsgemisch enthielt 30 ug Enzym, 50 mM Tris-HCl bei pH 7,5, 1 mM $NAD^+$ und 1 mM Substrat bei 30 °C. Die Reaktion wurde durch Zugabe des Enzyms gestartet. Das Reduktionsreaktionsgemisch enthielt 500 ug Enzym, 50 mM Citrat-Phosphat-Puffer bei pH 5,0, 1 mM NADH und 1 mM Substrat bei 30 °C für die Enzymanalyse. Für das Screening von manipulierten Enzymvarianten wurden zusätzlich 12,5 mM Formiat und 1 mg Formiatdehydrogenase zugegeben. Die Reaktion wurde durch Zugabe von BDH-Enzym gestartet. Zur Bestimmung des pH-Optimums wurde das Enzym in dem entsprechenden Puffer entsalzt und die Aktivität wie beschrieben gemessen. Das Temperaturoptimum wurde durch Inkubation der Testlösungen vor und während der Reaktion bei der entsprechenden Temperatur bestimmt. Die kinetische Analyse wurde bei optimalen pH- und Temperaturbedingungen durchgeführt (pH 5,0 und 30°C). Die $K_m$ und Kcat von $NAD^+$ wurden in Gegenwart von 1 mM Substrat und 0,01, 0,02, 0,04, 0,08, 0,1, 0,2, 0,4, 0,6, 0,8, 1 mM Cofaktor bestimmt. Die $K_m$ und Kcat von $NAD^+$ für (+)-Borneol und (-)-Borneol wurden in Gegenwart von 1 mM $NAD^+$ und 0,02, 0,04, 0,08, 0,1, 0,2, 0,4, 0,6, 0,8, 1 und 2 mM Substrat bestimmt. Für die Datenanalyse wurde das Enzymkinetikmodul von SigmaPlot v13.0 verwendet.

c) Racematspaltung (enantioselektive Reduktion von Campher zu Borneol)

(Verfahrensschritte a) bis c))

Das Reaktionsgemisch enthielt 50 mM Citrat-Phosphat Puffer pH 5,0, 500 μg BDH, 12,5 mM Glukose, 17 U/ml Glukosedehydrogenase, 0,05 mM NADH und 2 mM Campherracemat. Die Reaktion wurde durch Zugabe der BDH gestartet. Das Gemisch wurde bei 30 °C unter Schütteln inkubiert. Nach 135 min war der 1R-(+)-Campher komplett umgesetzt.

d) Nachweis der (+)-Borneol-Dehydrogenase-Aktivität

Ein spektrophotometrisches Verfahren wurde verwendet, um die Aktivität der Borneol-Dehydrogenase nachzuweisen. Die Reaktion wurde durch Zugabe von $NAD^+$ initiiert. Das Oxidationsreaktionsgemisch enthielt 30 ug Enzym, 50 mM Tris-HCl bei pH 7,5, 1 mM $NAD^+$ und 1 mM Substrat bei 30 °C. Die Reaktion wurde durch Zugabe des Enzyms gestartet und die NADH-Bildungsrate bei 340 nm photometrisch verfolgt.

e) Bestimmung der Campher-Reduktase-Aktivität des (+)-BDH-Enzyms

Das Reduktionsreaktionsgemisch enthielt 500 ug Enzym, 50 mM Citrat-Phosphat-Puffer bei pH 5,0, 1 mM NADH und 1 mM Substrat bei 30 °C für die Enzymanalyse. Für das Screening von manipulierten Enzymvarianten wurden zusätzlich 12,5 mM Formiat und 1 mg Formiatdehydrogenase zugegeben. Die Reaktion wurde durch Zugabe von Borneol-Dehydrogenase-Enzym gestartet und für 24 h inkubiert.

Tabelle 2: Kinetische Racematspaltung von Campher, katalysiert durch den Wild-Typ der Borneol-Dehydrogenase sowie die erfindungsgemäßen enantioselektiven (+)-Borneol-Dehydrogenasen.

| BDH Variante | $ee_S$ | $ee_P$ | Umsatz (+)-Campher | E(+) |
|---|---|---|---|---|
| WILDTYP | 24 % | 9 % | 27 % | 1,7 |
| BDH_S146A | 8 % | 71 % | 11 % | 6,3 |
| BDH_E145G | 6 % | 71 % | 8 % | 6,3 |
| BDH_E145H | 4 % | 71 % | 5 % | 6,0 |
| BDH_E145M | 27 % | 68 % | 29 % | 6,9 |
| BDH_E145L | 30 % | 67 % | 31 % | 6,6 |
| BDH_E145S | 59 % | 59 % | 50 % | 7,0 |
| BDH_Y188L | 4 % | 71 % | 6 % | 6,2 |
| BDH_Y188I | 9 % | 74 % | 11 % | 7,3 |
| BDH_Y188V | 17 % | 82 % | 17 % | 12,1 |
| BDH_Y188T | 28 % | 98 % | 22 % | >100 |
| BDH_Y188A | 29 % | 99 % | 23 % | >100 |

In Tabelle 2 sind Ergebnisse einer erfindungsgemäßen Racematspaltung gemäß der Verfahrensschritte a) bis c) dargestellt unter Verwendung der Wildtyp-Borneol-Dehydrogenase sowie der erfindungsgemäßen (+)-Borneol-Dehydrogenasen aufweisend einen Aminosäure-Austausch der Aminosäure S146 mit A, oder der Aminosäure E145 mit einer Aminosäure ausgewählt aus G, H, M, L, und S, oder der Aminosäure Y188 mit einer Aminosäure ausgewählt aus L, I, V, T und A. Erkenntlich sind die gemäß Beispiel 2c) und e) bestimmten und jeweils zugehörigen Enantiomeren-Anreicherungen $ee_s$ und $ee_p$, Umsätze (bezogen auf racemischen Campher) und (+)-Enantiomeren-Verhältnisse E(+). Dabei weist die Wildtyp-Borneol-Dehydrogenase einen Umsatz von 27 % und ein (+)-Enantiomeren-Verhältnis von 1,7 (E(+)) auf. Die ebenfalls dargestellten Versuchsergebnisse der 11 erfindungsgemäßen (+)-Borneol-Dehydrogenasen aufweisend einen Aminosäure-Austausch weisen Umsätze und (+)-Enantiomeren-Verhältnisse von mindestens 5 % und mindestens 6 (E(+)) auf. Die erfindungsgemäßen enantioselektiven (+)-Borneol-Dehydrogenasen weisen daher einen signifikanten Umsatz und eine signifikante (+)-Enantioselektivität auf. Die erfindungsgemäßen enantioselektiven (+)-Borneol-Dehydrogenasen BDH_E145M, BDH_E145L, BDH_E145S weisen eine gegenüber dem Wildtyp erhöhten Umsatz bei gleichzeitig ausgeprägter (+)-Enantioselektivität auf. Die erfindungsgemäßen enantioselektiven (+)-Borneol-Dehydrogenasen BDH_Y188V, BDH_Y188T, BDH_Y188A, insbesondere BDH_Y188T und BDH_Y188A, weisen bei signifikantem Umsatz gegenüber dem Wildtyp deutlich erhöhte (+)-Enantioselektivitäten auf. Demgemäß weisen die erfindungsgemäßen enantioselektiven (+)-Borneol-Dehydrogenasen aufweisend einen Aminosäure-Austausch in jedem Fall ein erhöhtes (+)-Enantiomeren-Verhältnis von mindestens 6 (E(+)) bis mehr als 100 (E(+)) gegenüber der Wildtyp-Borneol-Dehydrogenase auf.

Tabelle 3: Kinetische Racematspaltung von Campher, katalysiert durch den Wild-Typ der Borneol-Dehydrogenase sowie die erfindungsgemäßen enantioselektiven (+)-Borneol-Dehydrogenasen.

| BDH Variante | $ee_S$ | $ee_P$ | Umsatz (+)-Campher | E(+) |
|---|---|---|---|---|
| WILDTYP | 24 % | 9 % | 27 % | 1,7 |
| BDH_E145M/Y188T | 11 % | 97 % | 10 % | 72,6 |
| BDH_E145M/Y188A | 2 % | 94 % | 2 % | 30,5 |
| BDH_E145L/Y188T | 19 % | 100 % | 16 % | >100 |
| BDH_E145L/Y188A | 12 % | 99 % | 11 % | >100 |
| BDH_E145S/Y188T | 5 % | 96 % | 5 % | 50,0 |
| BDH_E145S/Y188A | 20 % | 100 % | 16 % | >100 |
| BDH_S146A/Y188T | 65 % | 97 % | 40 % | >100 |
| BDH_S146A/Y188A | 64 % | 99 % | 39 % | >100 |

In Tabelle 3 sind Ergebnisse einer erfindungsgemäßen Racematspaltung gemäß der Verfahrensschritte a) bis c) dargestellt unter Verwendung der Wildtyp der erfindungsgemäßen Borneol-Dehydrogenase sowie der erfindungsgemäßen (+)-Borneol-Dehydrogenasen aufweisend zwei Aminosäure-Austausche der Aminosäure E145 mit einer Aminosäure ausgewählt aus M, L und S und der Aminosäure Y188 mit einer Aminosäure ausgewählt aus T und A oder der Aminosäure S146 mit A und der Aminosäure Y188 mit einer Aminosäure ausgewählt aus T und A der Aminosäure . Erkenntlich sind die gemäß Beispiel 2c) und e) bestimmten und jeweils zugehörigen Enantiomeren-Anreicherungen $ee_s$ und $ee_p$, Umsätze (bezogen auf racemischen Campher) und (+)-Enantiomeren-Verhältnisse E(+). Dabei weist die Wildtyp-Borneol-Dehydrogenase einen Umsatz von 27 % und ein (+)-Enantiomeren-Verhältnis von 1,7 (E(+)) auf. Die ebenfalls dargestellten Versuchsergebnisse der 8 erfindungsgemäßen (+)-Borneol-Dehydrogenasen aufweisend zwei Aminosäure-Austausche weisen Umsätze und (+)-Enantiomeren-Verhältnisse von mindestens 2 % und mindestens 30,5 (E(+)) auf. Die erfindungsgemäßen enantioselektiven Borneol-Dehydrogenasen weisen daher einen Umsatz, insbesondere signifikanten Umsatz und eine signifikante (+)-Enantioselektivität auf. Die erfindungsgemäßen enantioselektiven (+)-Borneol-Dehydrogenasen BDH_E145M/Y188T, BDH_E145M/Y188A, BDH_E145L/Y188T, BDH_E145L/Y188A, BDH_E145S/Y188T und BDH_E145S/Y188A weisen eine gegenüber dem Wildtyp erheblich erhöhte (+)-Enantioselektivität auf. Die erfindungsgemäßen enantioselektiven (+)-Borneol-Dehydrogenasen BDH_S 146A/Y188T und BDH_S 146A/Y188A weisen zu dem Wildtyp einen deutlich erhöhten Umsatz und deutlich erhöhte (+)-Enantioselektivitäten auf. Demgemäß bilden die erfindungsgemäßen enantioselektiven (+)-Borneol-Dehydrogenasen aufweisend zwei Aminosäure-Austausche in jedem Fall ein erhöhtes (+)-Enantiomeren-Verhältnis von mindestens 30,5 (E(+)) bis mehr als 100 (E(+)) gegenüber der Wildtyp-Borneol-Dehydrogenase aus.

Tabelle 4: Kinetische Analyse des nicht-enantioselektiven Wildtyps sowie der erfindungsgemäßen enantioselektiven (+)-Borneol-Dehydrogenasen BDH_S146A/Y188T und BDH_S146A/Y188A.

| BDH variante | $K_m$ (–)-Borneol [mM] | $K_m$ (+)-Borneol [mM] | $K_{Cat}$ (–)-Borneol [$sec^{-1}$] | $K_{Cat}$ (+)-Borneol [$sec^{-1}$] |
|---|---|---|---|---|
| **WILDTYP** | **0,13** | **0,08** | **0,705** | **0,488** |
| **BDH_S146A/Y188 T** | **0,28** | **0,33** | **0,061** | **1,97** |
| **BDH_S146A/Y188 A** | **1,28** | **0,45** | **0,148** | **3,59** |

$K_m$ = Michealis-Konstante; $K_{Cat}$ = Geschwindigkeitskonstante des geschwindigkeits-bestimmenden Schritts, mM = millimolar. $sec^{-1}$ = 1/Sekunde

In Tabelle 4 sind Ergebnisse von kinetischen Enzymanalysen der Wildtyp der Borneol-Dehydrogenase sowie von zwei besonders bevorzugten erfindungsgemäßen (+)-Borneol-Dehydrogenase aufweisend zwei Aminosäure-Austausche der Aminosäure S146 mit A und der Aminosäure Y188 mit T sowie der Aminosäure S146 mit A und der Aminosäure Y188 mit A dargestellt. Erkenntlich sind die jeweiligen gemäß Beispiel 2b) bestimmten Michaelis-Konstanten $K_m$ und Reaktionsgeschwindigkeiten $K_{Cat}$. Dabei weist der Wildtyp eine $K_m$ von 0,13 bezogen auf (-)-Borneol und 0,08 bezogen (+)-Borneol sowie eine Kcat von 0,705 bezogen auf (-)-Borneol und 0,488 bezogen auf (+)-Borneol auf. Die zwei besonders bevorzugten erfindungsgemäßen (+)-Borneol-Dehydrogenasen aufweisend zwei Aminosäure-Austausche der Aminosäure S146 mit A und der Aminosäure Y188 mit T sowie der Aminosäure S146 mit A und der Aminosäure Y188 mit A weisen $K_m$ von 0,28 und 1,28 bezogen auf (-)-Borneol und 0,33 und 0,45 bezogen (+)-Borneol sowie eine Kcat von 0,061 und 0,148 bezogen auf (-)-Borneol und 1,97 und 3,59 bezogen auf (+)-Borneol auf und zeigen somit eine erhöhte (+)-Enantioselektivität gegenüber der Oxidation des (+)-Borneol-Enantiomers.

## Patentansprüche

1. Verfahren zur Herstellung einer (1S)-(-)-Campher-Enantiomeren-angereicherten Zusammensetzung aus Campher, umfassend die folgenden Verfahrensschritte:

   a) Bereitstellen von Campher, einem Reaktionsmedium und einer enantioselektiven (+)-Borneol-Dehydrogenase,
   b) Umsetzen des in Verfahrensschritt a) bereitgestellten Camphers in dem Reaktionsmedium mit der enantioselektiven (+)-Borneol-Dehydrogenase und
   c) Erhalten einer (1S)-(-)-Campher-Enantiomeren-angereicherten Zusammensetzung umfassend 1S-(-)-Campher und (+)-Borneol.

2. Verfahren nach Anspruch 1, wobei der in Verfahrens schritt a) bereitgestellte Campher racemisch ist.

3. Verfahren nach Anspruch 1, wobei der in Verfahrensschritt a) bereitgestellte Campher ein einen Enantiomeren-Überschuss von 1R-(+)-Campher oder 1S-(-)-Campher aufweisendes Gemisch ist.

4. Verfahren nach einem der Ansprüche 1 bis 3, wobei die in Verfahrensschritt a) bereitgestellte enantioselektive Borneol-Dehydrogenase enantioselektiv 1R-(+)-Campher umsetzt.

5. Verfahren nach einem der Ansprüche 1 bis 4, wobei die in Verfahrensschritt a) bereitgestellte enantioselektive Borneol-Dehydrogenase pflanzlichen oder mikrobiellen Ursprungs, bevorzugt mikrobiellen Ursprungs, oder synthetisch hergestellt ist.

6. Verfahren nach einem der Ansprüche 1 bis 5, wobei das Umsetzen in Verfahrensschritt b) unter Anwesenheit mindestens eines reduzierenden Co-Faktors durchgeführt wird, insbesondere NADH oder NADPH, bevorzugt NADH, oder einem NADH/NADPH-Analogon.

7. Verfahren nach einem der Ansprüche 1 bis 6, wobei das in dem in Verfahrensschritt c) erhaltene Gemisch aus 1S-(-)-Campher und (+)-Borneol in einem Verfahrensschritt d) mittels selektiver chemischer Umsetzung oder einer physikalischen Auftrennmethode in mindestens eine Campher- und eine Borneol-haltige Fraktion aufgetrennt wird, insbesondere mit mindestens einer Chromatographie- oder Destillationsmethode oder Kristallisation.

8. Verfahren nach einem der Ansprüche 1 bis 7, wobei das in Verfahrens schritt d) erhaltene (+)-Borneol in einem Verfahrensschritt e) zu 1R-(+)-Campher umgesetzt wird.

9. Verfahren nach einem der Ansprüche 1 bis 6, wobei die aus den Verfahrensschritten a) bis c) erhaltene (1S)-(-)-Campher-Enantiomeren-angereicherte Zusammensetzung in einem Verfahrensschritt f) zu 1S-(+)-Campher-10-Sulfonsäure umgesetzt wird, wobei der Verfahrensschritt f) folgende Schritte f1) bis f4) umfasst:

f1) Zugeben von Schwefelsäure zu der (1S)-(-)-Campher-Enantiomeren-angereicherten Zusammensetzung in einem organischen Lösungsmittel, insbesondere Acetanhydrid,
f2) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
f3) Erhalten einer Zusammensetzung von 1S-(+)-Campher-10-Sulfonsäure und (+)-Borneol, wobei vorzugsweise anschließend erfolgt ein
f4) Auftrennen der erhaltenen Zusammensetzung von 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

10. Verfahren nach Anspruch 9, wobei das in Verfahrensschritt f4) erhaltene (+)-Borneol in einem Verfahrensschritt g) zu 1R-(+)-Campher umgesetzt wird.

11. Verfahren nach einem der Ansprüche 1 bis 10, wobei das aus den Verfahrensschritten d), e) oder g) erhaltene 1R-(+)-Campher oder 1S-(-)-Campher in einem Verfahrensschritt h) zu 1R-(-)-Campher-10-Sulfonsäure oder 1S-(+)-Campher-10-Sulfonsäure umgesetzt wird, wobei der Verfahrensschritt h) folgende Schritte h1) bis h3) umfasst:

h1) Zugeben von Schwefelsäure zu 1R-(+)-Campher oder (1S)-(-)-Campher in einem organischen Lösungsmittel, insbesondere Acetanhydrid,
h2) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
h3) Erhalten von 1R-(-)-Campher-10-Sulfonsäure oder 1S-(+)-Campher-10-Sulfonsäure.

12. Verfahren zur Herstellung von 1S-(+)-Campher-10-Sulfonsäure umfassend die folgenden Verfahrensschritte i1) bis i5):

i1) Durchführen der Verfahrensschritte gemäß Anspruch 1 Verfahrensschritte a) bis c),
i2) Zugeben von Schwefelsäure zu der (1S)-(-)-Campher-Enantiomeren-angereicherten Zusammensetzung in einem organischen Lösungsmittel, insbesondere Acetanhydrid,
i3) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
i4) Erhalten einer Zusammensetzung von 1S-(+)-Campher-10-Sulfonsäure und (+)-Borneol, wobei vorzugsweise anschließend erfolgt ein
i5) Auftrennen der erhaltenen Zusammensetzung von 1S-(+)-Campher-10-Sulfonsäure und (+)-Borneol mittels selektiver chemischer Umsetzung oder einer physikalischen Auftrennmethode in mindestens eine Campher-10-Sulfonsäure-haltige und eine Borneol-haltige Fraktion, insbesondere mit mindestens einer Chromatographie- oder Destillationsmethode oder Kristallisation.

**13.** Verfahren nach Anspruch 12, wobei das aus Verfahrensschritt i5) erhaltene (+)-Borneol in einem Verfahrensschritt j) zu 1R-(+)-Campher umgesetzt wird.

**14.** Verfahren zur Herstellung von 1R-(-)-Campher-10-Sulfonsäure oder 1S-(+)-Campher-10-Sulfonsäure umfassend die folgenden Verfahrensschritte k1) bis k4):

k1) Durchführen der Verfahrensschritte nach Anspruch 7 Verfahrensschritte a) bis d), oder Anspruch 8 Verfahrensschritte a) bis e), oder Anspruch 10 Verfahrensschritte a) bis g), oder Anspruch 13 Verfahrensschritte a) bis j),
k2) Zugeben von Schwefelsäure zu dem aus Verfahrensschritt d), e), g) oder j) erhaltenen 1R-(+)-Campher oder (1S)-(-)-Campher in einem organischen Lösungsmittel, vorzugsweise Acetanhydrid,
k3) Umsetzen des erhaltenen Gemischs bei einer Temperatur von -20 °C bis 22 °C, insbesondere 0 °C bis 20 °C und über einen Zeitraum von 10 h bis 100 h, insbesondere 30 h bis 40 h und
k4) Erhalten von 1R-(-)-Campher-10-Sulfonsäure oder 1S-(+)-Campher-10-Sulfonsäure.

**15.** Enantioselektive Borneol-Dehydrogenase umfassend mindestens einen Aminosäure-Austausch mindestens einer Aminosäure mit einer der fünf Funktionalitäten polar, unpolar, phenolisch, sauer oder basisch der Wildtyp-Borneol-Dehydrogenase gemäß SEQ ID No. 1, **dadurch gekennzeichnet, dass** die ausgetauschte Aminosäure durch eine Aminosäure mit einer der vier anderen Funktionalitäten ersetzt wird.

**16.** Enantioselektive Borneol-Dehydrogenase gemäß Anspruch 15, wobei die mindestens eine in der SEQ ID No. 1 ausgetauschte Aminosäure ausgewählt ist aus der Gruppe bestehend aus I18, V92, G94, V95, Q96, R97, H98, S99, L112, N116, F142, G143, S144, E145, S146, G147, L148, T149, G150, E151, I152,D153, N154, G155, L156, Y157, A158, A159, T160, K161, A162, H165, V185, L186, P187, Y188, M189, V190, T191, L201, S202, P203, A205, L206, A207, K214, D216, I217, F223 und L230 (jeweils gemäß SEQ ID No. 1).

**17.** Enantioselektive Borneol-Dehydrogenase gemäß einem der Ansprüche 15 oder 16, wobei die mindestens eine in der SEQ ID No. 1 ausgetauschte Aminosäure ausgewählt ist aus der Gruppe bestehend aus I18, V95, Q96, R97, H98, S144, E145, S146, E151, I152,D153, N154, Y157, A158, K161, P187, Y188, M189, T191 und S202 (jeweils gemäß SEQ ID No. 1).

**18.** Enantioselektive Borneol-Dehydrogenase gemäß einem der Ansprüche 15 bis 17, wobei die mindestens eine in der SEQ ID No. 1 ausgetauschte Aminosäure in einer Aminosäuresequenz im Bereich von Q96 bis Y188 liegt, insbesondere ausgewählt ist aus der Gruppe bestehend aus Q96, H98, E145, S146, N154 und Y188 (jeweils gemäß SEQ ID No. 1).

**19.** Enantioselektive Borneol-Dehydrogenase gemäß einem der Ansprüche 15 bis 18, wobei diese einen oder zwei Aminosäure-Austausche aufweist, wobei wenn ein Aminosäure-Austausch vorliegt, dieser insbesondere an einer der Positionen E145, S146 oder Y188 erfolgt, oder wobei wenn zwei Aminosäure-Austausche vorliegen, diese an Positionen S146 und Y188 oder E145 und Y188 erfolgen (jeweils gemäß SEQ ID No. 1).

**20.** Enantioselektive Borneol-Dehydrogenase gemäß einem der Ansprüche 15 bis 19, wobei die Aminosäure E145 ausgetauscht ist durch H, M, L, S oder G (jeweils gemäß SEQ ID No. 1).

**21.** Enantioselektive Borneol-Dehydrogenase gemäß einem der Ansprüche 15 bis 20, wobei die Aminosäure Y188 ausgetauscht ist durch T, A, V, I oder L (jeweils gemäß SEQ ID No. 1).

**22.** Enantioselektive Borneol-Dehydrogenase gemäß einem der Ansprüche 15 bis 21, wobei die Aminosäure S146 ausgetauscht ist durch A (gemäß SEQ ID No. 1).

**23.** Enantioselektive Borneol-Dehydrogenase gemäß einem der Ansprüche 15 bis 22, wobei die Borneol-Dehydrogenase eine Enantioselektivität von bevorzugt mindestens 10 und/oder einen Umsatz von bevorzugt mindestens 10 % (bezogen auf racemischen Campher) aufweist.

**24.** Polynukleotid, kodierend eine enantioselektive Borneol-Dehydrogenase gemäß einem der Ansprüche 15 bis 23.

**25.** Vektoren, enthaltend ein Polynukleotid nach Anspruch 24.

**26.** Wirtszelle, enthaltend einen Vektor nach Anspruch 25.

**27.** Verfahren zur Herstellung einer enantioselektiven Borneol-Dehydrogenase gemäß einem der Ansprüche 15 bis 23, umfassend die folgenden Verfahrensschritte:

l) Bereitstellen einer Wirtszelle gemäß Anspruch 26,
m) Kultivieren der in Verfahrensschritt 1) bereitgestellten Wirtszelle in einem Kulturmedium unter Bedingungen, die die Expression einer enantioselektiven Borneol-Dehydrogenase gemäß einem der Ansprüche 15 bis 23 ermöglichen und
n) Erhalten der in Verfahrensschritt m) exprimierten enantioselektiven Borneol-Dehydrogenase.

**Figur 1**

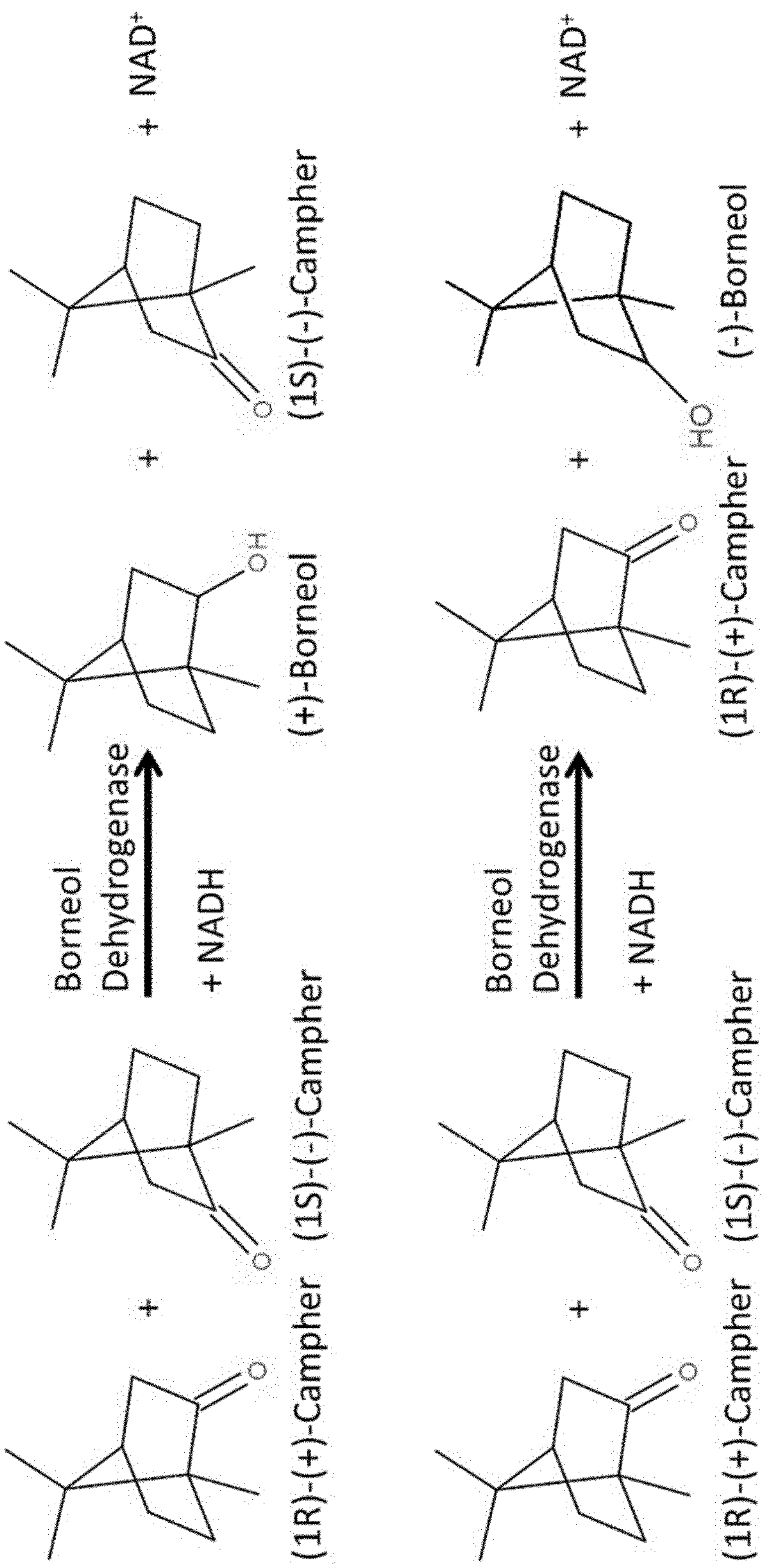
(1R)-(+)-Campher
+
(1S)-(-)-Campher
Borneol Dehydrogenase
+ NADH
(+)-Borneol
+
(1S)-(-)-Campher
+ NAD+
(1R)-(+)-Campher
+
(1S)-(-)-Campher
Borneol Dehydrogenase
+ NADH
(1R)-(+)-Campher
+
(-)-Borneol
+ NAD+

**Figur 2**

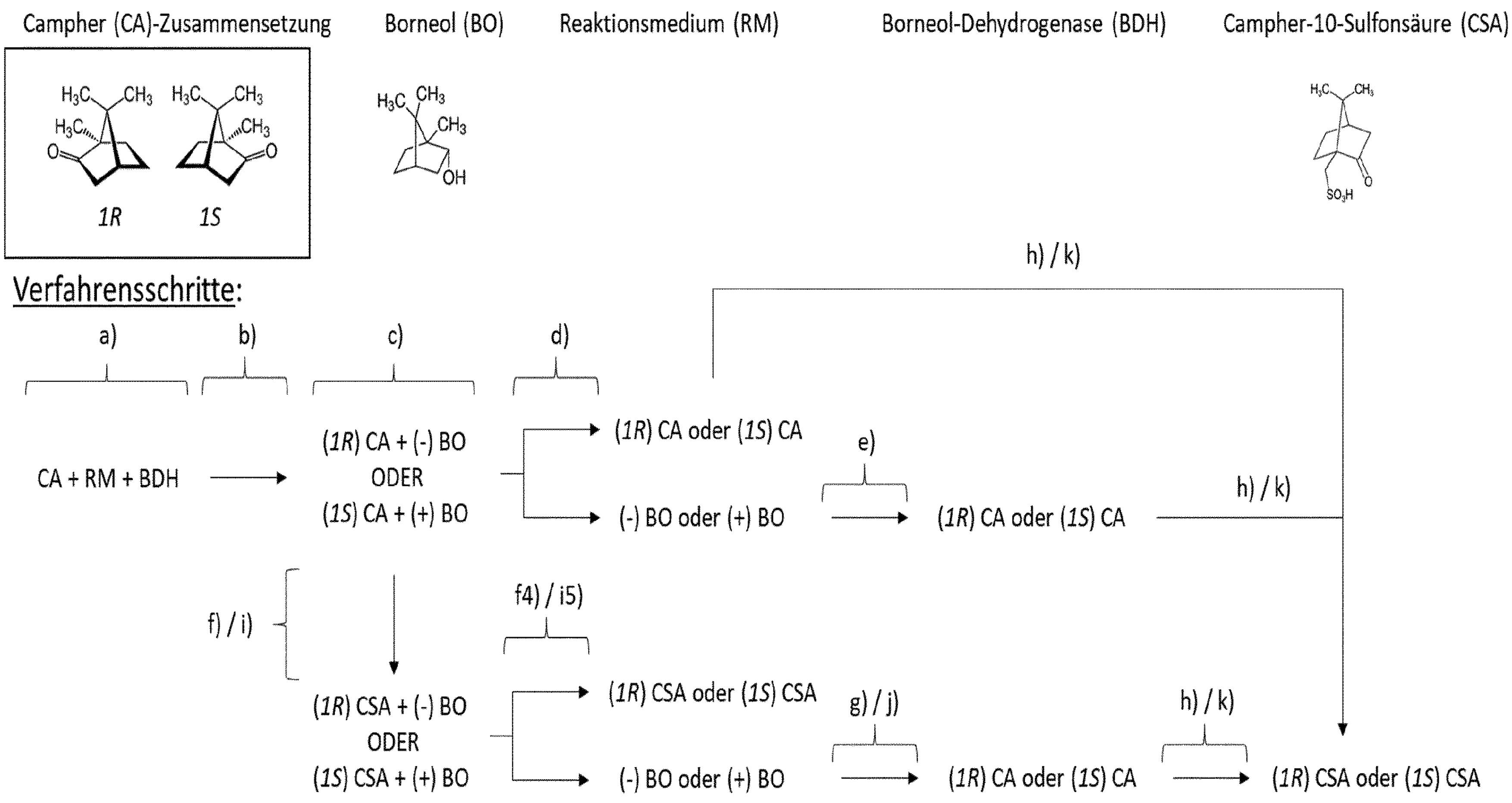
Campher (CA)-Zusammensetzung
1R
1S
Borneol (BO)
Reaktionsmedium (RM)
Borneol-Dehydrogenase (BDH)
Campher-10-Sulfonsäure (CSA)
Verfahrensschritte:
a)
b)
c)
d)
CA + RM + BDH
(1R) CA + (-) BO
ODER
(1S) CA + (+) BO
(1R) CA oder (1S) CA
(-) BO oder (+) BO
e)
(1R) CA oder (1S) CA
h) / k)
h) / k)
f) / i)
f4) / i5)
(1R) CSA + (-) BO
ODER
(1S) CSA + (+) BO
(1R) CSA oder (1S) CSA
(-) BO oder (+) BO
g) / j)
(1R) CA oder (1S) CA
h) / k)
(1R) CSA oder (1S) CSA

Figur 3

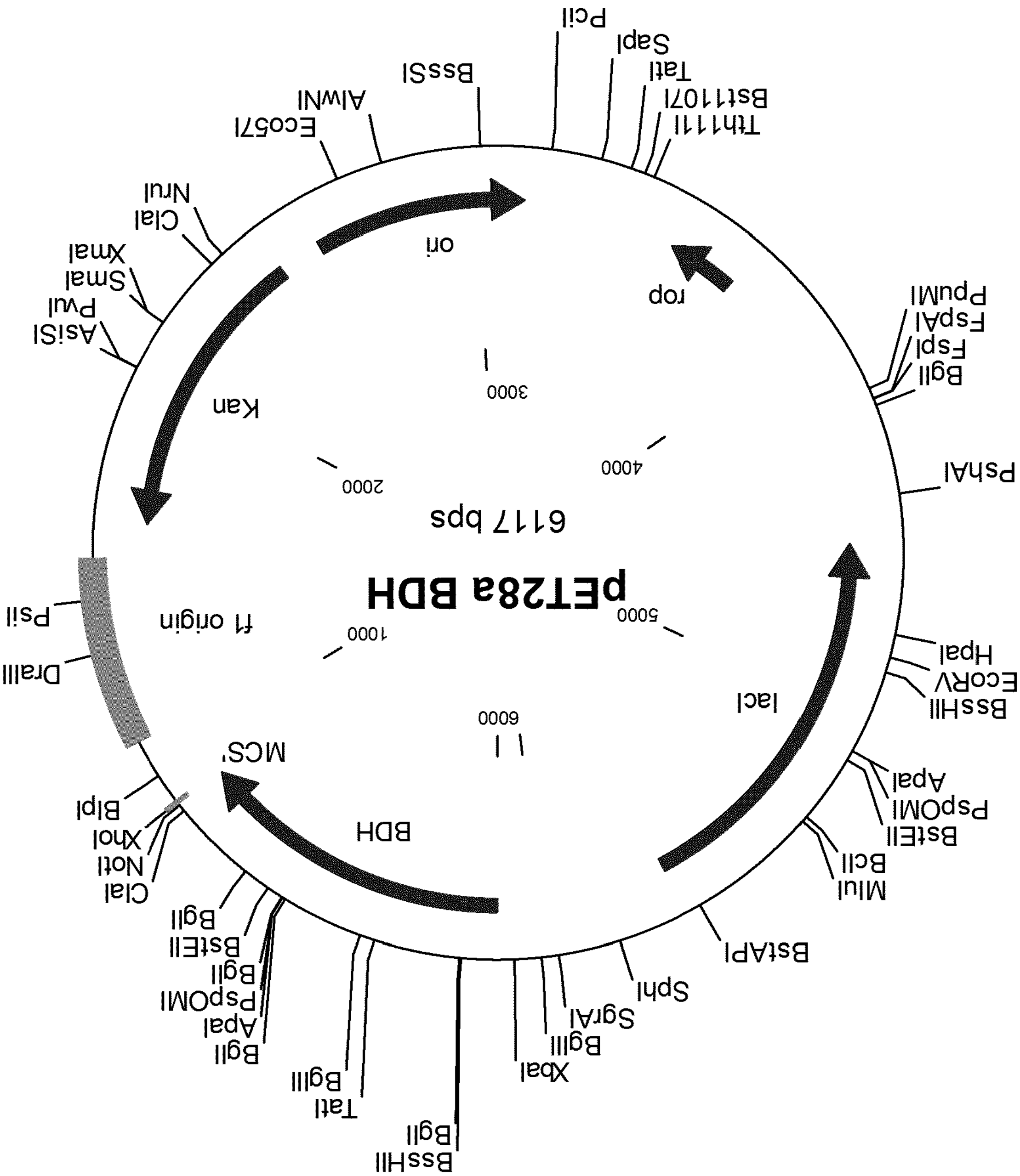
pET28a BDH
6117 bps
1000
2000
3000
4000
5000
6000
ori
rop
Kan
f1 origin
lacI
BDH
MCS'
PciI
SapI
TatI
Bst1107I
Tth111I
BssSI
AlwNI
Eco57I
NruI
ClaI
XmaI
SmaI
PvuI
AsiSI
PpuMI
FspAI
FspI
BglI
PshAI
HpaI
EcoRV
BssHII
ApaI
PspOMI
BstEII
BclI
MluI
BstAPI
SphI
SgrAI
BglII
XbaI
BglI
BssHII
TatI
BglII
BglI
ApaI
PspOMI
BglI
BstEII
BglI
ClaI
NotI
XhoI
BlpI
DraIII
PsiI

Europäisches Patentamt
European Patent Office
Office européen des brevets

# EUROPÄISCHER RECHERCHENBERICHT

Nummer der Anmeldung
EP 21 20 4928

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | HOI-LUNG TSANG ET AL: "Borneol Dehydrogenase from Pseudomonas sp. Strain TCU-HL1 Catalyzes the Oxidation of (+)-Borneol and Its Isomers to Camphor", APPLIED AND ENVIRONMENTAL MICROBIOLOGY, Bd. 82, Nr. 21, 19. August 2016 (2016-08-19), Seiten 6378-6385, XP055486067, Washington DOI: 10.1128/AEM.01789-16 * das ganze Dokument * ----- | 1-27 | INV. C12P41/00 C12N9/04 C12R1/40 |
| A | DATABASE Uniprot [Online] 11. Dezember 2019 (2019-12-11), Leisch Hannes ET AL: "Putative short-chain dehydrogenase/reductase SDR of Pseudomonas putida ATCC 17453", XP055903698, Database accession no. M5AW56 * Zusammenfassung * ----- | 1-27 | |
| | | | RECHERCHIERTE SACHGEBIETE (IPC) |
| A | BRINDA PRASAD ET AL: "Identification of Camphor Oxidation and Reduction Products in: New Activity of the Cytochrome P450System", JOURNAL OF CHEMICAL ECOLOGY, KLUWER ACADEMIC PUBLISHERS-PLENUM PUBLISHERS, NE, Bd. 37, Nr. 6, 12. Mai 2011 (2011-05-12), Seiten 657-667, XP019913684, ISSN: 1573-1561, DOI: 10.1007/S10886-011-9959-7 * das ganze Dokument * ----- | 1-27 | C12P C12N C12R |

-/--

Der vorliegende Recherchenbericht wurde für alle Patentansprüche erstellt

1

| Recherchenort | Abschlußdatum der Recherche | Prüfer |
|---|---|---|
| Berlin | 23. März 2022 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 21 20 4928

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DRIENOVSKÁ IVANA ET AL: "Molecular cloning and functional characterization of a two highly stereoselective borneol dehydrogenases from Salvia officinalis L", PHYTOCHEMISTRY, ELSEVIER, AMSTERDAM , NL, Bd. 172, 9. Januar 2020 (2020-01-09), XP086064199, ISSN: 0031-9422, DOI: 10.1016/J.PHYTOCHEM.2019.112227 [gefunden am 2020-01-09] * das ganze Dokument * ----- | 1-27 | |
| X,P | HOFER MICHAEL ET AL: "Engineering of a borneol dehydrogenase from P. putida for the enzymatic resolution of camphor", APPLIED MICROBIOLOGY AND BIOTECHNOLOGY, SPRINGER BERLIN HEIDELBERG, BERLIN/HEIDELBERG, Bd. 105, Nr. 8, 1. April 2021 (2021-04-01) , Seiten 3159-3167, XP037427153, ISSN: 0175-7598, DOI: 10.1007/S00253-021-11239-5 [gefunden am 2021-04-12] * das ganze Dokument * ----- | 1-27 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Berlin | 23. März 2022 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3819689 A
- IN 151660 A1


### In der Beschreibung aufgeführte Nicht-Patentliteratur


- **BANTHORPE et al.** *Sogo Kango,* 1972, vol. 72, 115-155
- **BRADSHAW et al.** *JACS,* 1959, vol. 81, 788-824
- **ZHEN-DONG ; LIANG-WU.** *Chemical Engineering,* 2009, vol. 43, 1-8
- **XU et al.** *Neurosci,* 2005, vol. 25, 8924-8937
- **SELESCU et al.** *Chem. Sense,* 2013, vol. 38, 563-575
- **SHERKHELI et al.** *Pak. J.. Pharm. Sci.,* 2013, vol. 26, 431-438
- **BREUER et al.** *Angew. Chem. Int.,* 2004, vol. 43, 788-824
- **VERHO ; BACKVALL.** *JACS,* 2015, vol. 137, 3996-4006
- **HOFER et al.** *ChemCatChem,* 2013, vol. 5, 3351-3357
- **DEHAL, CROTEAU.** *Arch. Biochem. Biophys.,* 1987, vol. 258, 287-291
- **DRIENOVSKÁ et al.** *Phytochemistry,* 2020, vol. 172, 112227
- **TSANG et al.** *Appl. Environ. Microbiol.,* 2016, vol. 82, 6378-6385
- **EDELHEIT et al.** *BMC Biotechnol.,* 2009, vol. 9, 61